# EUROPEAN PATENT SPECIFICATION

(11) **EP 2 442 845 B1**
(45) Date of publication and mention of the grant of the patent: **21.08.2013**
(21) Application number: 10755203.6
(22) Date of filing: 15.06.2010
(51) Int. Cl.: A61M 1/16

(54) **DIALYSIS MACHINE**
DIALYSEMASCHINE
APPAREIL DE DIALYSE

(30) Priority: 15.06.2009 GB 0910244; 15.06.2009 GB 0910246
(43) Date of publication of application: 25.04.2012
(73) Proprietor: Quanta Fluid Solutions Ltd, Warwickshire B49 6EU (GB)
(72) Inventor: WALLACE, Mark, South Staffordshire DY7 6EL (GB); HIGGIT, Ben, Birmingham B47 5RD (GB); HEYES, Keith, James, Worcestershire B45 8NR (GB)
(74) Representative: Croston, David
(86) International application number: PCT/GB2010/001161
(87) International publication number: WO 2010/146343

(56) References cited:
- EP-A1- 0 623 357
- WO-A2-2005/089832
- WO-A2-2008/106191
- US-B1- 6 228 047

## Description

The present invention relates to dialysis machines and in particular, but not exclusively, to a disposable cartridge for use in a hemodialysis machine.

Dialysis is a treatment which replaces the renal function of removing excess fluid and waste products, such as potassium and urea, from blood. The treatment is either employed when renal function has deteriorated to an extent that uremic syndrome becomes a threat to the body's physiology (acute renal failure) or, when a longstanding renal condition impairs the performance of the kidneys (chronic renal failure).

There are two major types of dialysis, namely hemodialysis and peritoneal dialysis.

In peritoneal dialysis treatment, a dialysate solution is run through a tube into the peritoneal cavity. The fluid is left in the cavity for a period of time in order to absorb the waste products, and is subsequently removed through the tube for disposal.

It is common for patients in the early stages of treatment for a longstanding renal condition to be treated by peritoneal dialysis before progressing to hemodialysis at a later stage.

In hemodialysis, the patient's blood is removed from the body by an arterial line, is treated by the dialysis machine, and is then returned to the body by a venous line. The machine passes the blood through a dialyser containing tubes formed from a semi permeable membrane. On the exterior of the semi permeable membrane is a dialysate solution. The semi permeable membrane filters the waste products and excess fluid from the blood into the dialysate solution. The membrane allows the waste and a controlled volume of fluid to permeate into the dialysate whilst preventing the loss of larger more desirable molecules, like blood cells and certain proteins and polypeptides.

The action of dialysis across the membrane is achieved primarily by a combination of diffusion (the migration of molecules by random motion from a region of higher concentration to a region of lower concentration), and convection (solute movement that results from bulk movement of solvent, usually in response to differences in hydrostatic pressure).

Fluid removal (otherwise known as ultrafiltration) is achieved by altering the hydrostatic pressure of the dialysate side of the membrane, causing free water to move across the membrane along the pressure gradient.

The correction of uremic acidosis of the blood is achieved by use of a bicarbonate buffer. The bicarbonate buffer also allows the correction of the blood bicarbonate level.

The dialysis solution consists of a sterilized solution of mineral ions. These ions are contained within an acid buffer which is mixed with water and bicarbonate base prior to delivery to the dialyser. The water used is cleaned to a sufficient degree that it is suitable for use as a base for trans-membrane ion transfer with the blood (hereinafter sterile water); this may for example be achieved by known methods including reverse osmosis, heat treatment, filtration or a combination of such known methods.

Dialysate composition is critical to successful dialysis treatment since the level of dialytic exchange across the membrane, and thus the possibility to restore adequate body electrolytic concentrations and acid-base equilibrium, depends on the composition.

The correct composition is accomplished primarily by formulating a dialysate whose constituent concentrations are set to approximate normal values in the body.

However, achieving the correct composition of dialysate requires the accurate control of low volumes of liquid and at present this is achieved by the provision of complex fluid paths, including multiple pumping and valving components on the dialysis machine.

This presents the disadvantage of a complex and costly dialysis machine which is at increased risk of failure by virtue of its complexity. Increased maintenance is also a problem since it is essential to minimise machine downtime in order to most efficiently treat the patient.

A further problem with known hemodialysis machines is that the blood and dialysate solution lines require careful mounting onto the dialysis machine before the treatment can commence. This presents a risk that the lines are not correctly installed, a risk which is particularly relevant to those patients who dialyse at home.

This method of dialysis also presents an increased risk of cross-infection between patients since the disposable blood and dialysate lines come into contact with the dialysis machine.

The problems associated with conventional dialysis equipment are mitigated to some degree by the system disclosed in WO 2006/120415 which discloses a cartridge based system for conducting hemodialysis, however the method and system for mixing the dialysate proposed in this application is complex and costly involving a large cartridge with multiple reservoirs, each having level control and therefore requiring a complex pumping and control system. Both this complexity and this space requirement are undesirable in portable dialysis machines, for example those suitable for home dialysis. A further problem associated with this design is that the overflow ports associated with the reservoirs present a possible route for bacteria to get into the dialysate. Another system of this kind is disclosed in document WO2008/106191, yet using a mixing reservoir.

It is an object of the present invention to provide a hemodialysis system which at least mitigates some of the problems described above.

According to a first aspect of the invention there is provided a cartridge for use in a hemodialysis machine, the cartridge comprising:
a dialysate flow path including a dialyser, the dialysate flow path for delivering a flow of dialysate to the dialyser;
a first mixing pump comprising a chamber having a fixed volume between a recess and a flexible membrane, said chamber for receiving a predetermined volume of a first dialysate solution base, and a volume of water;
a flow balancing pump comprising: a chamber having a fixed volume between a recess and a flexible membrane, and an inlet through which it receives dialysate; and
a fluid flow path connecting the first mixing pump to the flow balancing pump;
   the cartridge further comprising:
a first check valve having an inlet in fluid communication with said fluid flow path, said check valve configured to open if the pressure in the fluid flow path exceeds a predetermined pressure to allow excess fluid in the fluid flow path to flow through the first check valve to a drain, and to close when the pressure in the fluid flow path falls back below said predetermined pressure.

The first check valve allows for any excess in volume of first dialysate solution base and water to bleed out of the system, this feature enables the balancing chamber to completely fill on each and every stroke enabling accurate flow balancing. This reduces the complexity of the control system needed to drive the cartridge as inaccuracies or variations in the sizes of the various chambers are compensated by allowing some of the fluid to bleed out of the check valves.

Preferably the cartridge comprises a blood flow path for carrying a volume of blood to be treated in the dialyser.

Preferably the cartridge is disposable.

Preferably the cartridge according further comprises:
a second mixing pump downstream of the first mixing pump, the second mixing pump comprising a chamber having a fixed volume between a recess and a flexible membrane for receiving a predetermined volume of a second dialysate solution base and the mixture of water and first dialysate solution base from the first mixing pump, and wherein
the inlet of the first check valve is in fluid communication with the fluid flow path between said first and second mixing pumps.

Preferably the cartridge further comprises:
a second check valve having an inlet, said second check valve inlet in fluid communication with the fluid flow path between the second mixing pump and the balancing pump, wherein
the second check valve is configured to open if the pressure in the fluid flow path between the second mixing pump and the balancing pump increases above a predetermined pressure to allow excess fluid in the fluid flow path to flow through the second check valve to a drain, and to close when the pressure in the fluid flow path falls back below the said predetermined pressure.

In this manner a two part dialysate can be mixed with water in two mixing pumps, the excess from each mixing pump being allowed to bleed to drain.

In a preferred arrangement the chamber of the second mixing pump and the chamber of the flow balancing pump are one and the same chamber.

In this way one of the chambers can be eliminated thereby reducing the complexity of the control system necessary to drive the dialysate mixing, and also a smaller cartridge can be affected.

In a preferred arrangement the cartridge further comprising:
a second mixing pump downstream of the first mixing pump, the second mixing pump comprising a chamber having a fixed volume between a recess and a flexible membrane, said chamber for receiving a predetermined volume of a second dialysate solution base and the mixture of water and first dialysate solution base from the first mixing pump, and wherein
the inlet of the first check valve is in fluid communication with the fluid flow path between the second mixing pump and the flow balancing pump.

Preferably the second mixing pump has a volume greater than the first mixing pump.

In this manner, when two mixing pumps and a balancing pump are used the drain between the first and second mixing pump is eliminated as the second pump is preferably made sufficiently large that it can always accept all of the fluid from the chamber of the first mixing pump.

In a preferred arrangement the cartridge further comprises:
a first dialysate base pump, said first dialysate base pump configured to add a volume of the first dialysate base to the first mixing pump, and
a controller to control the first dialysate base pump.

Preferably the cartridge further comprises:
a first sensor in the fluid flow path, said sensor downstream of the first mixing pump, the sensor arranged to feedback a signal indicative of the concentration of the mixture of first dialysate solution base and water exiting the first mixing pump, to the controller, and wherein
the controller is configured to control the first dialysate solution base pump to achieve a predetermined ratiometric mix of first dialysate solution base and water.

Preferably the cartridge further comprises:
a first dialysate solution base sensor that creates a signal indicative of the concentration of the first dialysate solution base, and wherein
the controller receives said signal and uses it to control the first dialysate solution base pump to achieve a predetermined ratiometric mix of first dialysate solution base and water in the chamber of the first mixing pump.

The signal may either be used to monitor the concentration or may be used to monitor for an error, such as disruption of the supply of first dialysate base solution.

Preferably the cartridge further comprises:
a second dialysate base pump configured to add a volume of the second dialysate base to the second mixing pump, and
a controller to control the second dialysate base pump.

Preferably the cartridge a further comprises:
a sensor downstream of the second mixing pump that creates a signal indicative of the concentration of the mixture of second dialysate solution base and the mixture of water and first dialysate solution base exiting the second mixing pump, and wherein
the controller receives said signal and uses it to control the second dialysate solution base pump to achieve a predetermined ratiometric mix of second dialysate solution base with the mixture of water and first dialysate solution.

Preferably the cartridge further comprises:
a second dialysate solution base sensor that creates a signal indicative of the concentration of the second dialysate solution base, and wherein
the controller receives said signal and uses it to control the second dialysate solution base pump to achieve a predetermined ratiometric mix of second dialysate solution base with the mixture of water and first dialysate solution in the chamber of the second mixing pump.

According to a second aspect of the invention there is provided a cartridge for use in a hemodialysis machine, the cartridge comprising:
a dialysate flow path including a dialyser, the dialysate flow path for delivering a flow of dialysate to the dialyser;
a first mixing pump comprising a chamber having a fixed volume between a recess and a flexible membrane, said chamber for receiving a predetermined volume of a first dialysate solution base, and a volume of water;
a first dialysate solution base pump, controlled by a controller, for adding a predetermined volume of a first dialysate solution base to the first mixing pump chamber;
a flow balancing pump comprising a chamber having a fixed volume between a recess and a flexible membrane and an inlet through which it receives dialysate; and
a fluid flow path connecting the first mixing pump to the flow balancing pump;
wherein the minimum toleranced volume of the first mixing pump is equal to, or greater than, the maximum toleranced volume of the balancing pump.

In this manner it can be guaranteed that, even taking into consideration variations in volume due to manufacturing tolerance, the volume of dialysate flowing from the first mixing pump to the balancing pump will always be sufficient to completely fill the balancing pump chamber. Any volume of fluid in the chamber of the first mixing pump not received in the chamber of the balancing pump will remain as residual fluid in the first mixing pump.

Preferably the cartridge includes a blood flow path for carrying a volume of blood to be treated in the dialyser.

Preferably the cartridge is a disposable.

In one preferred arrangement the cartridge further comprises:
a second dialysate mixing pump upstream of the first dialysate mixing pump, the second mixing pump comprising a chamber having a fixed volume between a recess and a flexible membrane, said chamber for receiving a predetermined volume of a second dialysate solution base, and a volume of water;
a second dialysate solution base pump, controlled by a controller, for adding a predetermined volume of a second dialysate solution base to the second mixing pump chamber; and
a second fluid flow path connecting the second and the first mixing pumps for delivering a mixture of water and second dialysate base solution to the chamber of the first mixing pump.

Preferably the minimum toleranced volume of the second mixing pump is equal to, or greater than, the maximum toleranced volume of the first mixing pump.

In this manner it can be guaranteed that, even taking into consideration variations in volume due to manufacturing tolerance, the volume of dialysate flowing from the second mixing pump to the first mixing pump will always be sufficient to completely fill the first mixing pump chamber. Any volume of fluid in the chamber of the second mixing pump not received in the chamber of the first mixing pump will remain as residual fluid in the second mixing pump.

Preferably the cartridge further comprises a sensor downstream of the first and/or the second mixing pumps, said sensor configured to create a signal indicative of the concentration of the fluid exiting each of the first and/or second mixing pumps.

In another alternative preferred arrangement the balancing pump is configured to receive into its chamber: a mixture of water and first dialysate base solution from the first mixing point, and a second dialysate solution base solution.

Preferably the cartridge further comprises a sensor downstream of the mixing pump, said sensor configured to create a signal indicative of the concentration of the fluid exiting the balancing pump.

In one preferred arrangement, in use, feedback signals from the sensors are fed to respective controllers of the first and second dialysate solution base pumps, the respective controllers configured to control the first and second dialysate solution base pumps to modify the amount of first and second dialysate solution base they add to the first and second mixing pumps, or first mixing pump and balancing pump, respectively, so as to achieve a predetermined ratiometric mixture of first and second dialysate solution base and water.

Measuring the concentration and feeding back the signal enables the concentration of the diluted first and/or second dialysate base solution to be constantly checked and furthermore enables the amount introduced into the chambers to be altered to compensate for any drift or changes over time.

In another preferred arrangement, in use, signals from the sensors are monitored and cause an alarm if the signal generated falls outside of specific parameters. In this mode of operation the sensors are used for safety monitoring and can stop the system if the dialysate mix is not correct.

According to a third aspect of the invention there is provided a method of mixing a dialysate solution in the cartridge according to the second aspect of the invention, the method comprising:
filling the chamber of the first mixing pump with a predetermined volume of first dialysate base solution and water;
pumping a proportion of the mixture in the chamber of the first mixing pump into the chamber of the balancing pump; and
retaining a proportion of the mixture of first dialysate base solution and water in the chamber of the first mixing pump.

In this manner the mixing of the dialysate on the cartridge can be effected with a simplified control circuit as, due to retaining the excess volume of fluid in the first pump chamber the chambers will come to a natural equilibrium.

Preferably the method further comprises:
measuring a property of the dialysate base solution and water exiting the first mixing chamber to generate a signal indicative of its concentration,
determining if the concentration is greater or lesser than the required concentration;
controlling the first dialysate solution base pump to increase or decrease the amount of first dialysate solution base added to the chamber of the first mixing pump accordingly, and repeating the method until the required concentration is achieved.

Preferably the method further comprises:
filling the chamber of the second mixing pump with a predetermined volume of second dialysate base solution and water;
pumping a proportion of the mixture of second dialysate base solution and water, and a predetermined volume of first dialysate solution base, into the chamber of the first mixing pump;
retaining a proportion of the mixture of second dialysate base solution and water in the chamber of the second mixing pump.

Preferably the method further comprises:
measuring a property of the second dialysate base solution and water exiting the second mixing chamber to generate a signal indicative of its concentration,
determining if the concentration is greater or lesser than the required concentration;
controlling the second dialysate solution base pump to increase or decrease the amount of second dialysate solution base added to the chamber of the second mixing pump accordingly.

Preferably the method comprises repeating the steps of filling and partially emptying the chambers until equilibrium is achieved and the required dialysate concentrations are achieved.

The invention will now be described, by way of example only, and with reference to the following drawings, in which:
Figures 1 to 10 show a dialysis machine and cartridge according to the prior art; and
Figures 11 to 15 are schematic diagrams for the dialysate preparation flow paths of the invention.

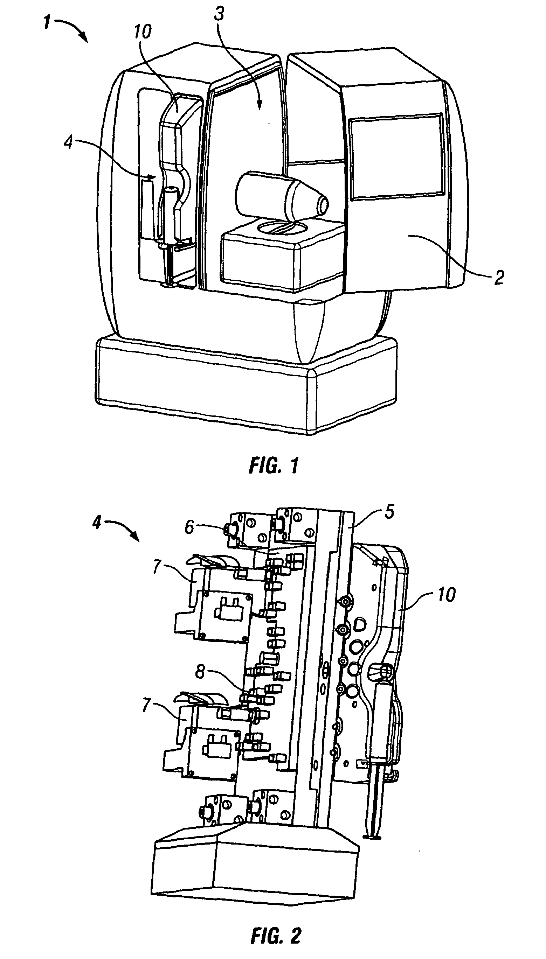

In Figure 1 a dialysis machine 1 is shown having a cover 2 which opens to reveal a storage compartment 3. The machine has an engine section 4 which receives a dialysis cartridge 10.

Referring now to Figure 2, the engine section 4 is shown in further detail to include first and second platens 5, 6 which close upon insertion of the cartridge 10 into the machine to retain the cartridge in position in use. The engine 4 has pneumatic actuators 7 and sensors (indicated generally at 8 in Figure 2) arranged on the second platen to control operation of the cartridge 10 as will be described in further detail shortly.

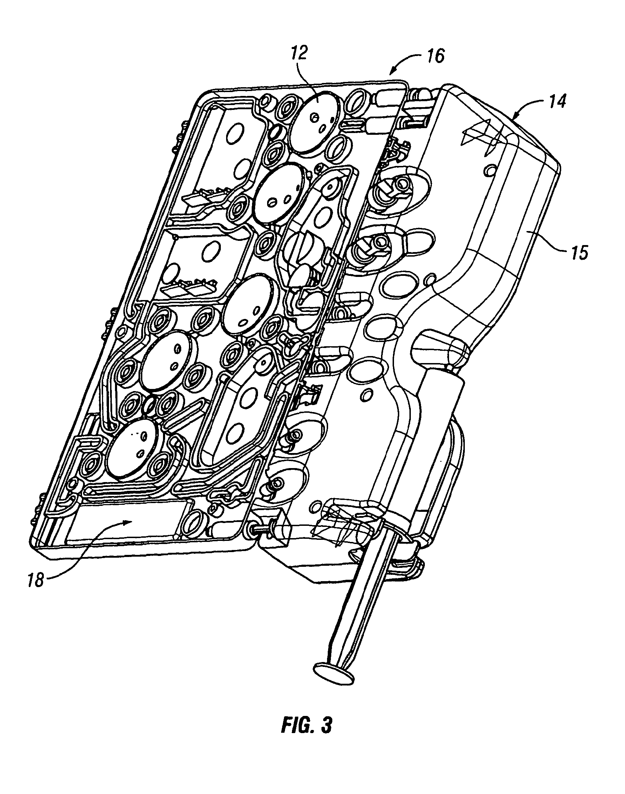

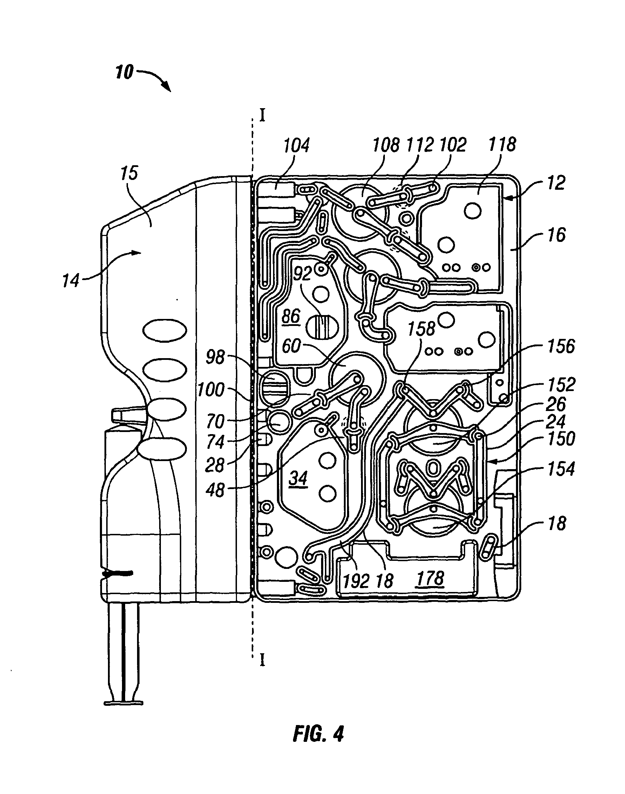

In figures 3 and 4 a dialysis cartridge 10 is shown having a pumping portion 12 (to the right of dashed line I-I in figure 4) and a dialysis portion 14 (to the left of dashed line I-I in figure 4). The pumping portion 12 has the form of a flat rectangle. The dialysis portion 14 has a dialyser cover 15.

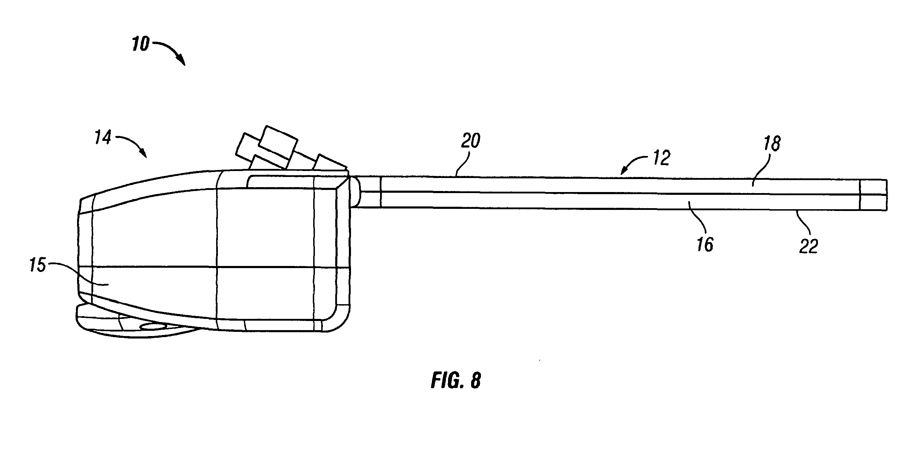

Referring briefly to Figure 8, the pumping portion 12 of the dialysis cartridge 10 has an upper surface 16 and a lower surface 18. The upper surface 16 and a lower surface 18 are covered by a clear membrane 20, 22, respectively, which is formed from a deformable plastics material. The first and second membrane, 20, 22 are bonded to the upper surface 16 and a lower surface 18, respectively by way of adhesive or similar known method.

Referring now to Figure 4, the upper surface 16 defines a series of upstanding walls indicated, for example, as 24. The upstanding walls 24 define a system of flow channels as will be described in further detail shortly. The channels are enclosed at the outermost part of the upper surface 16, by the first membrane 20. Accordingly, the upper surface 16 defines a series of fluid channels for carrying either the blood to be dialysed, or the Dialysate solution.

The cartridge 10 also defines the series of apertures, indicated generally for example at 26 in Figure 4. These apertures provide a fluid pathway through the cartridge 10, the purpose of which will now be described.

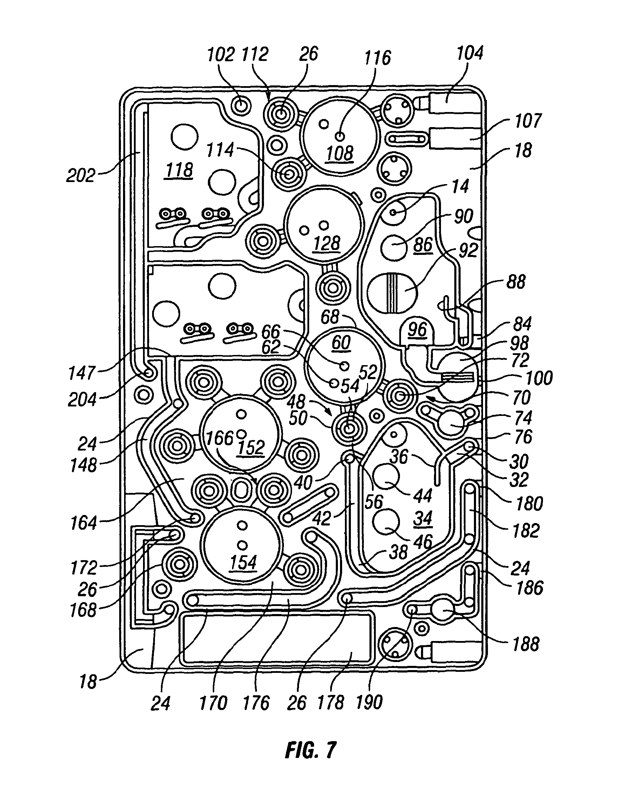

Referring to Figure 7, the lower surface 18 also defines a series of upstanding walls 24, which collectively define a labyrinth of fluid channels enclosed by the second membrane 22.

In combination therefore the upper surface 16, lower surface 18 and the first and second membranes 20, 22 form a series of interconnected fluid flow paths on both sides of the pumping portion 12. This labyrinth of fluid flowing pathways will now be described in further detail.

The first membrane 20 is bonded to the upper surface 16, and similarly the second membrane 22 bonded to the lower surface 18, so as to contain the fluids within their respective channels.

The dialyser cartridge 10 defines two primary fluid pathways, firstly, a flow path for blood and secondly a flow path for the dialysate solution. The blood pathway is formed as follows.

The patient's blood enters the dialysis cartridge 10 via an arterial port 28. The blood then passes from the upper surface 16 to the lower surface 18 via an arterial port aperture 30 where it is then carried by an arterial port channel 32 from the arterial aperture 30 to an arterial blood bubble trap 34. The arterial blood bubble trap 34 has an inlet lip 36 for directing the incoming blood towards the bottom of the trap. Arranged at the bottom of the trap is a blood bubble trap exit 38 which carries the blood from the arterial blood bubble trap 34 to an arterial blood bubble trap aperture 40 via channel 42.

The purpose of the arterial blood bubble trap 34 is to remove from the arterial blood supply any gas bubbles which may be contained therein. Gas bubbles may impair the performance of dialyser, and furthermore, present a risk to the patient if they were reintroduced back into the body via the venous blood line. The blood bubble trap 34 is also provided with an upper level sensor port 44 and a lower level sensor port 46. The level sensor ports 44, 46 are arranged to coincide with corresponding optical level sensors arranged on the dialysis machine. Accordingly, the level sensors are able to optically interrogate the arterial blood bubble trap 34 so as to ensure that the level in the blood bubble trap is above the level of the lower level sensor port 46 and below the level of the upper level sensor port 44. It is important to ensure that the blood level remains between these two levels so that there always remains a volume of air in the blood level trap into which any gas bubbles carried in the blood can migrate.

Having passed through the arterial blood bubble trap aperture 40 the blood is carried on the upper surface 16 to a blood pump inlet valve 48 (see Figure 4).

Referring to Figure 4, the blood pump inlet valve 48 is operable between a closed condition and an open condition as follows. The valve 48 has an outer annular upstanding wall 50 and an inner annular upstanding wall 52. Arranged inwardly of the inner upstanding annular wall 52 is a valve aperture 54. The inner upstanding annular wall 52 is recessed from the outer upstanding annular wall 50 in a direction towards the cartridge 10. Arranged between the inner and outer upstanding annular wall 50, 52 is a sector aperture 56 which acts as an outlet from the valve 48. Accordingly, the valve 48 has a valve inlet in the form of valve aperture 54 and an outlet in the form of the sector aperture 56. As discussed previously, the lower surface 18 has its outer service covered by a deformable membrane 22. The deformable membrane 22 rests against the outwardly facing surface of the outer upstanding annular wall 50 where the valve is in the unactuated, open state. In order to change the condition of the valve 48 from the open state to the closed state, the dialysis machine applies a positive pressure to the exterior surface of the second membrane 22 in order to drive the inner surface of the membrane on to the outwardly facing surface of the inner upstanding annular wall 50. This closes the inlet to the valve thereby preventing flow through the valve.

With the blood pump inlet valve 48 in the open state, the blood flows through the arterial blood bubble trap aperture 40 over the inner upwardly standing wall 50 and through the sector aperture 56 so as to exit the blood pump inlet valve 48. From the sector aperture 56 the blood then flows down a blood pump inlet channel 58 and into a blood pump 60 via a blood pump inlet 62.

The blood pump is defined by a dome shaped pump cavity 64 into which the blood pump inlet 62 opens. Arranged at the centre of the pump chamber 64 is a pump outlet 66. The outer edge of the pump chamber 64 is defined by an annular upstanding wall 68, the outwardly facing surface of which is in contact with the inner surface of the second membrane 22. A volume of blood is drawn into the pump chamber 64, through the open blood pump inlet valve 48 as follows.

The dialysis machine generates a negative pressure on the outside surface of the second membrane 22 in order to deform the membrane outwardly away from the lower surface 18. With the pump chamber 64 full, and the pump at full stroke, the blood pump inlet valve 48 is closed by the dialysis machine generating positive pressure on the outside surface of the second membrane 22 in order to close the valve aperture 54. The pump chamber 64 is then evacuated by the dialysis machine applying a positive pressure to the outside surface of the second membrane 22 in order to drive the blood contained within the pump chamber 64 through the pump outlet 66. The pump outlet 66 is in fluid communication with a blood pump outlet valve 70 which is identical in form to the blood pump inlet valve 48. It follows that with the blood pump inlet valve closed, and the blood pump 60 being driven by the dialysis machine to evacuate the pump 64, the blood pump outlet valve 70 is in an open state in order to permit the flow of blood past the valve 70 and through a blood pump outlet valve aperture 72.

Accordingly, the blood pump 60 is in combination with the blood pump inlet valve 48 and the blood pump outlet valve 70. Specifically, the blood pump inlet valve 48 opens when the blood pump is in the expansion stroke in order to admit blood into the pump chamber, whilst the blood pump outlet valve 70 remains closed in order to prevent back-flow of blood through the system. The inlet valve 48 then closes at the same time as the outlet valve 70 is opened in order to allow the compression stroke of the flow pump to drive the blood from the pump chamber 64 and through the blood pump outlet valve aperture 72.

From the aperture 72, the blood then flows through a pressure sensor chamber 74. As the blood flows through the chamber 74, the fluid pressure causes a force to be applied to the first membrane 20 which in turn causes a deflection in the membrane. This deflection is detected by a sensor provided in the dialysis machine and this measured deflection is calibrated to generate a blood pressure reading for within the cartridge.

From the pressure sensor chamber 74 the blood then passes through a dialyser blood port 76.

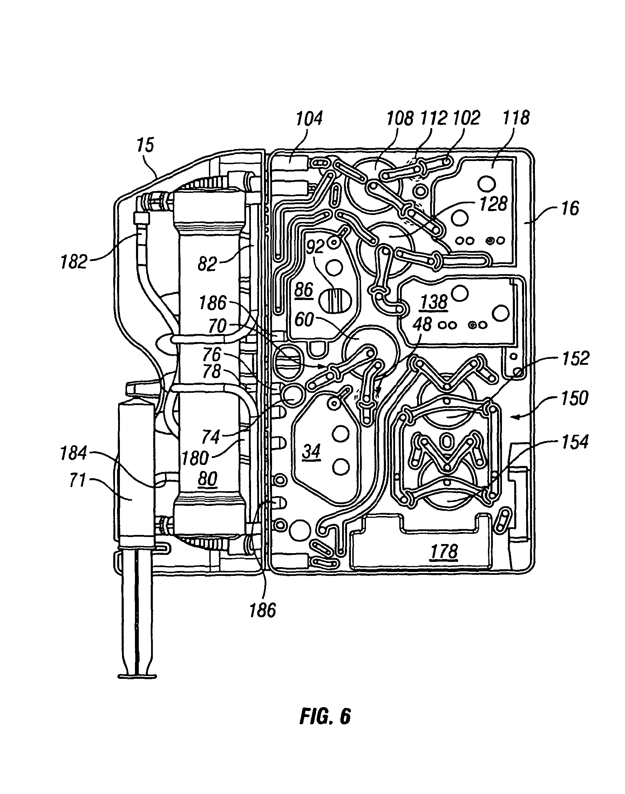

Referring now to Figure 6, the blood flows from the dialyser blood port 66 down a dialyser blood line 78 and into the bottom end of a dialyser 80 of known design. The dialyser 80 contains multiple axially extending semi-permeable tubes through which the blood passes. Upon exiting the dialyser 80 the blood travels down a dialyser return blood line 82 before passing into a venous blood bubble trap 86 via a dialyser blood return port 84.

The venous blood bubble trap 86 is similar in design to the arterial blood bubble trap 34 in that it has an inlet lip 88, an optical level sensor 90 and a hydrophilic membrane 94 to allow the hydrolysis machine withdraw or administer a volume of air to or from the bubble trap in order to maintain a constant blood level within the bubble trap. The venous blood level trap 86 is further provided with an ultrasonic level sensor 92 the design of which will be described in further detail shortly. At the bottom end of the valve trap is a thrombus filter 96 for trapping blood clots within the bubble trap. The Thrombus filter may be of conical form as in known thrombus filters or may be wedge shaped. Having passed through the thrombus filter 96, the blood passes through an ultrasonic flow rate sensor 98 which will be described in further detail shortly. The blood is then returned to the patient via a venous port 100.

The blood therefore completes its passage through the dialysis cartridge 10 from the arterial port 28 through the arterial blood bubble trap 34, the blood pump inlet valve 48 and into the blood pump 60. From blood pump 60 the blood is driven past the blood pump outlet valve 70 and into the dialyser 80 via the cross membrane pressure sensor 74. Upon exit from the dialyser 80, the blood is returned to the dialysis cartridge 10 via the dialyser blood return port 84. Upon exit from the port 84 the blood enters the venous blood bubble trap 86, passes through the thrombus filter 96 and flow sensor 98 before being returned to the patient via the venous port 100.

A syringe 71 is provided which introduces a volume of an anti-coagulant drug such as heparin into the blood line between the blood pump outlet valve 70 and the dialyser 80. The syringe plunger 73 is driven by the machine engine as shown in Figure 2.

As described above, dialysis occurs across a semi-permeable membrane, in this instance the semi-permeable tubes provided within the dialyser 80. As described, the blood flows through the centre of the semi-permeable tubes and it therefore follows that the dialysate solution flows in the space within the dialyser 80 between the tubes. The mixing of the dialysate solution on the cartridge at the correct concentration will now be described in detail.

The pump portion 12 defines the dialysate flow path in addition to the blood flow path as described above.

Accordingly, the dialysis cartridge 10 provides for the mixing into a sterile water supply of a small volume of concentrated bicarbonate solution and a small volume of acid solution. The resulting dialysate solution is pumped from the pumping portion to deliver the solution to the dialyser. The cartridge further allows for the accurate sensing of dialysate solution concentration, dialysate flow rate and dialysate pressure.

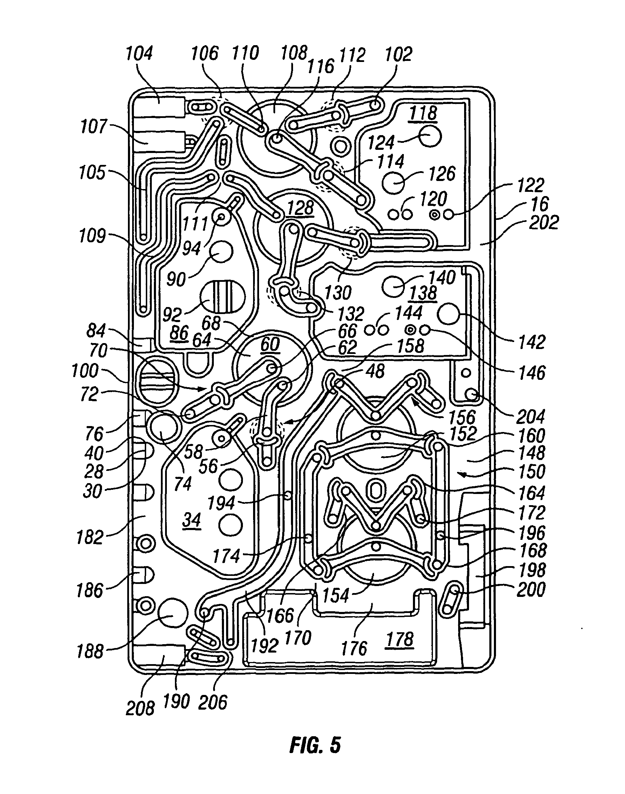

Sterile water enters the dialysis cartridge 10 via a sterile water inlet 102. The sterile water is then mixed with a controlled volume of bicarbonate solution base as follows. The cartridge 10 defines a chamber 104, for receiving the plunger of a positive displacement pump (not shown for clarity in Figures 3 to 9). The pump acts in combination with a three-way valve 106 of known design. The pump and three-way valve 106 are operated by the dialysis machine to micro-dose a controlled volume of bicarbonate solution into a bicarbonate pump 108. The bicarbonate pump 108 is of similar design to the blood pump 60 with the exception that the bicarbonate pump 108 is additionally provided with an inlet 110 from the three-way valve 106. The bicarbonate pump 108 is controlled in exactly the same manner to the flow pump 60 in order to draw a volume of sterile water through the sterile port at 102 and past a bicarbonate inlet pump 112 whilst a bicarbonate pump outlet valve 114 remains closed. At the same time as a volume of sterile water is drawn into the pump a small volume of saturated bicarbonate solution is injected into the bicarbonate pump 108 by a positive displacement pump. The body of the positive displacement pump is defined by the cartridge body. The saturated bicarbonate solution is drawn from a reservoir on the dialysis machine. The solution is delivered to the pump via a bicarbonate inlet channel 105 and three-way valve 106.

The action of drawing the water into the pump chamber by means of applying a negative pressure to the outer surface of the first membrane 20 generates a turbulent flow within the pump chamber which causes the sterile water and bicarbonate solution to be mixed thoroughly within the pump chamber. Accordingly, at the point where the bicarbonate pump inlet valve 112 is closed, and the outlet valve 114 opens in order to drive a solution from the pump chamber, a thorough homogeneous mixing has been achieved.

The bicarbonate and water solution is pumped out of the pump chamber via a pump exit 116 from which it flows past the pump outlet valve 114 and into a water-bicarbonate solution reservoir 118. The volume of the water-bicarbonate reservoir 118 is approximately four times the volume of the bicarbonate pump chamber and performs two functions. Firstly, it further ensures that the mixture is homogenous, and secondly acts as a fluid buffer within the dialysate solution flow path, the purpose of which will be described in further detail shortly.

The bicarbonate solution reservoir 118 is provided with a conductivity sensing probe 120 and a temperature sensing probe 122, an upper level sensor 124 and a lower level sensor 126.

The conductivity and temperature sensor probes are provided to contact with conductivity and temperature sensors in the dialysis machine. The measurements are used to deduce the concentration of the water-bicarbonate solution in the reservoir 118. The reservoir also acts as a buffer to allow for the various system pumps being out of phase. Accordingly, the level in the reservoir is able to rise and fall thereby averaging out pressure spikes in the system.

From the water-bicarbonate reservoir 118, the solution is drawn into an acid pump 128 past an open acid pump inlet valve 130. Coupled to the acid pump 128 is an acid pump outlet valve 132. The purpose of the acid pump 128 is to introduce a small volume of acid solution base into the water-bicarbonate solution. This process is achieved using the same valving and pumping methodology as employed for the bicarbonate pump 108. Specifically, a second chamber 107 is provided for receiving the plunger of a second positive displacement pump. A volume of acid solution base is thereby dispensed down an acid inlet channel 109 to a second 3-way valve 111. Under the action of the pump 128, water-bicarbonate solution is drawn into the pump chamber. The acid solution base is injected into the pump by a second positive displacement pump. The fluids are thoroughly mixed in the turbulent flow within the pump chamber before being dispensed past the outlet valve 132 into a water-bicarbonate-acid reservoir 138.

The water-bicarbonate-acid reservoir 138 is provided with a conductivity sensing probe 144 and a temperature sensing probe 146, an upper level sensor 140 and a lower level sensor 142, in common with the water-bicarbonate reservoir 118.

From the water-bicarbonate-acid reservoir 138, the solution flows through a reservoir exit 147 (see Figure 7) into a flow balance inlet channel 148. The solution is thereby delivered to the flow balancer 150.

The purpose of the flow balancer 150 is to ensure that the volume of dialysate solution pumped into the dialyser is the same as that withdrawn from the dialyser 80. The purpose of matching the flow into and out of the dialyser is to match the osmotic potential of the dialysate solution within the dialyser to the osmotic potential of the blood. This ensures that the volume of the fluid removed from the blood, or transferred to the blood, can be carefully controlled. This is critical to ensuring that the patient is not hydrated or dehydrated to a dangerous extent during the Dialysis treatment.

The flow balancer 150 is provided with a first flow balance pump 152 and a second flow balance pump 154. The first and second flow balance pumps 152, 154 have a similar mode of operation to the blood pump 60, and the mixing pumps 108, 128. However, the flow path for delivering fluid to each of the flow balance pumps 152, 154 is rather more complex due to the way in which the flow balancer 150 achieves the controlled fluid flow input and output from the dialyser 80.

In principal, the flow balancer 150 operates by using the first flow balance pump 152 to pump dialysate solution into the dialyser, and the second flow balance pump 154 to withdraw the dialysate solution from the dialyser, for a period of time, before switching the second flow balance pump 154 to pump dialysate solution into the dialyser, and the first flow balance pump 152 withdrawing dialysate solution from the dialyser. The purpose of this mode of operation is to eliminate the effect of manufacturing tolerances in generating a mismatch in the volume of the pump chamber in each of the flow balance pumps 152, 154. For example, were the first flow balance pump 152 used permanently to pump dialysate solution into the dialyser, and the second flow balance pump 154 used to withdraw dialysate solution from the dialyser, then over a period of time even the very small discrepancy in the pump chamber volume of the pumps would lead to a dangerous imbalance in the volume of dialysate solution being pumped into, and withdrawn from, the dialyser.

By switching the first and second flow balance pumps 152,154, any errors in the chamber volume are averaged over time, thereby ensuring a balance in the flow across the dialyser.

In selective fluid communication with the first flow balance pump 152 are a first flow balance pump first inlet valve 156, a first flow balance pump second inlet valve 158, a first flow balance pump first outlet valve 160 and a first flow balance pump second outlet valve 162. Similarly, in selectable fluid communication with the second flow balance pump 154 are a second flow balance pump first inlet valve 164, a second flow balance pump second inlet valve 166, a second flow balance pump first outlet valve 168 and a second flow balance pump second outlet valve 170.

The first mode of operation of the flow balancer 150 will now be described in detail. In the first mode of operation, the first flow balance pump first inlet valve 156, first flow balance pump second outlet valve 162, second flow balance pump second inlet valve 166 and second flow balance pump first outlet valve 168 are all held in the closed position by the dialysis machine applying a positive pressure to the outside surface of the first membrane 20 in the region of each of the valves. Accordingly, in the first mode of operation the second flow balance pump 154 is operated to pump dialysate solution into the dialyser, and the first flow balance pump 152 is operated to withdraw dialysate solution from the dialyser.

With the first flow balance pump first inlet valve 156 in the closed position, dialysate solution passing out of the bicarbonate acid reservoir 138 flows past the first flow balance pump first inlet valve 156 along a flow balance inlet channel 148. The dialysate solution then passes from the lower surface 18 to upper surface 16 via an aperture 172. With the second flow balance pump first inlet valve 164 in its open position, the second flow balance pump 154 is able to draw a volume of dialysate solution into the pump chamber under the action of the dialysis machine generating a negative pressure on the outward facing surface of the first membrane 20.

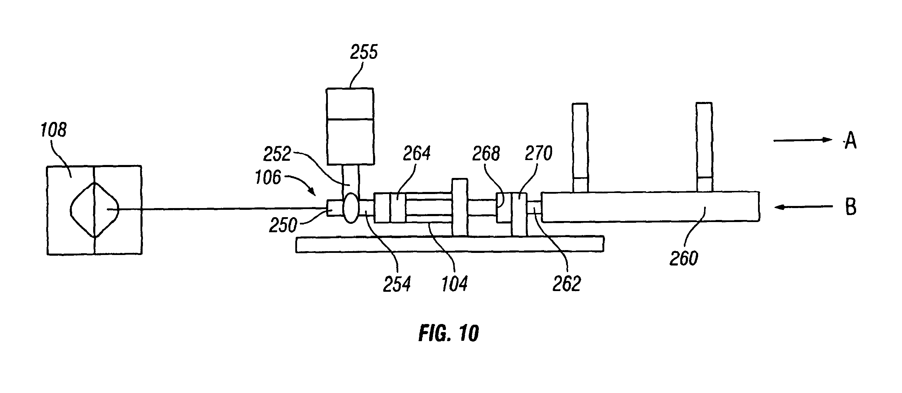

As soon as the second flow balance pump 154 is at full capacity, the second flow balance pump first inlet valve 164 is closed, and the second flow balance pump second outlet valve 170 is opened. The pump 154 is then actuated to discharge the dialysate solution through an aperture 174 and the dialysate solution then flows along channel 176 as shown in Figure 10. The dialysate solution then passes through an endotoxin filter 178 before passing through a dialyser output port 180 via channel 182.

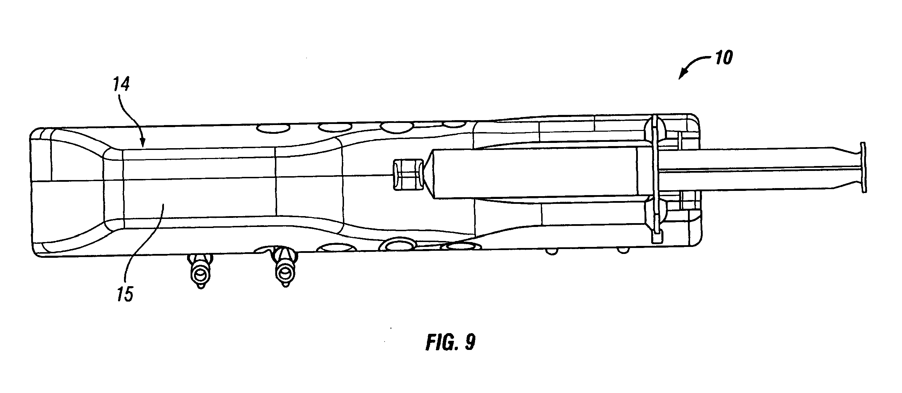

Referring now to Figure 6, from the dialysate outlet port 180, dialysate solution passes along a dialysate inlet pipe 180 before passing along the dialyser 80 from top to bottom as shown in Figure 9. In order to return the dialysate solution from the dialyser 80 to the pumping portion 12, a dialysate outlet pipe 184 carries dialysate solution to a dialysate inlet port 186. Upon return to the pumping portion 12, the dialysate solution passes through a colour sensor portion 188 in order to allow a colour sensor arranged on the dialysis machine to interrogate the dialysate solution to detect for blood leakage into the dialysate solution within the dialyser 80. On exit from the colour sensor portion 188, the dialysate solution passes through aperture 190 and from there into a flow balance return channel 192.

Since the second flow balance pump second inlet valve 166 is closed, the dialysate solution flows past aperture 194 towards the first flow balance pump second inlet valve 158. With the valve 158 in the open position, the first flow balance pump 152 is able to draw in to the pump chamber a volume of dialysate solution through the inlet valve 158 under the action of a positive pressure generated by the dialysis machine on the outwardly facing surface of the first membrane 20. The first flow balance pump second inlet valve 158 then closes, the first flow balance pump first outlet valve 160 opens, and the pump 152 drives the dialysate solution from the fluid chamber through the outlet valve 160. The outlet valve 160 is then closed, the inlet valve 158 opened and the pump 152 driven to draw in a further volume of dialysate solution ready for dispensing in the next pump cycle.

Having been delivered past the outlet valve 160, the dialysate solution flows through aperture 196 since the second flow balance pump first outlet valve 168 is closed during the mode of operation. The dialysate solution then passes through an ultrasonic flow sensor 198 which will be described in further detail shortly, before exiting the dialysis cartridge 10 by way of dialysate solution drain 200.

In the second mode of operation, the roles of the first and second flow balance pumps 152, 154 are reversed. In other words, the first flow balance pump second inlet valve 158 and first flow balance pump first outlet valve 160 are held closed whilst the first inlet valve 156 and second outlet valve 162 are operated to control the flow of Dialysate solution into and out of the valve chamber. Similarly, with reference to the second flow balance pump 154, the second flow balance pump first inlet valve 164 and second flow balance pump second outlet valve 170 are held in a closed position whilst the second flow balance pump second inlet valve 166 and second flow balance pump and first outlet valve 168 are operated to control the flow of the acid solution into and out of the pump chamber.

The technique of flow balancing, as described above, is provided to ensure that exactly the same volume of dialysate solution is pumped into the dialyser 80 as is removed from it. However, in certain dialysis treatments there is a requirement to either remove excess fluid from the blood, or to transfer fluid back into the blood. This is achieved by the process of ultra-filtration in which the flow balance circuit is placed slightly out of balance by either introducing or removing a small volume of liquid to or from the dialysate solution. In the dialysis cartridge of the present invention this is achieved by an ultra-filtration three-way valve 206 which acts in combination with a positive displacement pump received in chamber 208 on the cartridge. This combination of three-way valve and positive displacement pump is identical to that used to introduce the bicarbonate solution into the bicarbonate pump 108. The positive displacement pump plunger is received within the chamber 208 and is positioned by a drive, for example a stepper motor, on the dialysis machine.

The cartridge 10 has a drainage channel 202 for draining excess fluid from the water-bicarbonate reservoir 118 and the water-bicarbonate-acid reservoir 138. The drainage channel carries excess fluid from the reservoirs 118, 138 and dumps the fluid to drain via a drainage port 204 which is in fluid communication with a drainage port in the dialysis machine.

Accordingly, the dialysis cartridge 10 provides two distinct flow paths, firstly for blood, and secondly for dialysate solution. The provision of an upper surface 16 and a lower surface 18, with apertures therebetween allows the transfer of fluid from the outwardly facing surface of the upper surface to be on the facing surface of the lower surface. The blood flow path and the dialysate solution flow path are maintained discrete from one another by upstanding walls extending from the upper surface and lower surface. The outer surface of the upstanding walls abuts a deformable membrane in order to seal the flow path.

It will be appreciated that the apertures provided in the first and second cartridge bodies 16, 18 allow for the most convenient packaging of the various cartridge features. It is clear that this feature provides a distinct advantage over cartridges which define all of the flow channels on only one side of the cartridge.

In an alternative embodiment of cartridge, the arterial blood bubble trap 34 and venous blood bubble trap 86 have a collapsible element in the form of a concertina section of plastic material so as to limit the area of blood/air interface. This is particularly advantageous in that the reduced blood/air interface reduces the risk of clotting and/or separation of the blood.

A further alternative feature of the bubble trap is to replace the hydrophilic membrane 94 with a membrane pump similar to the blood pump 60. Accordingly, instead of air being added or removed to the bubble trap by way of the transfer of air across the hydrophilic membrane, the transfer of air can be achieved by the displacement of the membrane by the application of either a positive or negative pressure on the outwardly facing surface of the membrane. Furthermore, the extent of actuation of the membrane could be monitored in order to detect where an excessive volume of air is either being added to the reservoir or removed from the reservoir.

In a further alternative embodiment, each of the valves, for example 48, 106, 112, 114, 164 etc. are provided with rigid disks which have the diameter equal to or slightly greater than the diameter of the inner upwardly standing wall. The rigid disk is arranged between the inner upwardly standing wall and the membrane. The purpose of the rigid disc is to minimise the deformation required in the membrane in order to seal the valve. In other words, the membrane acts on the rigid disc which in turn forms a valve seat on the inner upwardly standing wall. The result of the reduced deformation of the membrane is that the transient shock waves generated in the valve by virtue of the switching between open and closed is reduced since the valve is closed at a lower peak pressure than would be necessary if the rigid disc were not present. A further benefit in addition to the reduction in pressure spiking observed in the valve body is the reduced blood damage achieved by smoother operation of the valve between its open and closed state.

Referring now to Figure 10, the positive displacement pump and three-way valve on the current invention are shown schematically in further detail. The three-way valve is indicated generally at 106. It will be appreciated that the three-way valve 106 is identical to the valve in communication with the pump 128 and the ultra-filtration valve 206. The detailed description of three-way valve 106 therefore applies equally to the other two three-way valves provided on the dialysis cartridge.

The bicarbonate mixing pump 108 is connected via a fluid line to an output 250 of the three-way valve 106. The three-way valve also has a reservoir inlet 252 and a pump inlet 254. The reservoir inlet 252 is connected to a bicarbonate solution reservoir 255. The reservoir 255 is provided on the dialysis machine, or attached thereto, and does not form part of the cartridge itself. The positive displacement pump is indicated generally at 258. The positive displacement pump includes a pneumatic cylinder 260 which drives a piston arm 262 in a reciprocating manner. At the opposite end of the piston arm to the piston cylinder is a plunger 264 which acts within the cartridge chamber 104 (see Figure 10).

On the return stroke indicated at A in Figure 10, the plunger 264 is moved within the chamber 104 to draw in to the chamber a measured volume of dialysate solution from the bicarbonate solution reservoir 255. This transfer of fluid is achieved by the closure of the three-way valve output 250, with the reservoir inlet and pump inlet 252, 254 remaining open. The piston arm 262 is withdrawn in direction A until an abutment 268 provided on the piston arm 262 comes into contact with a moveable end stop 270.

Upon the abutment 268 hitting the moveable end stop 270, the pneumatic cylinder 268 is driven in direction B in order to dispense the dialysate solution from the chamber 104 into the bicarbonate mixing pump 108. This transfer of fluids is achieved by the closure of the reservoir inlet 252, and the opening of the three-way valve output 250. The pneumatic cylinder 260 drives the piston ring 264 in direction B until the piston ring abuts the extreme left hand end of the chamber 104. Accordingly, by reciprocating the movement of the cylinder piston arm 262 in a known manner, a quantity of bicarbonate solution is repeatedly dispensed into the bicarbonate mixing pump 108. Furthermore, by adjusting the position of the removable end stop 270, the volume of fluid dispensed can be accurately set. The moveable end stop 270 is positioned by a stepper motor or similar accurate positioning drive system.

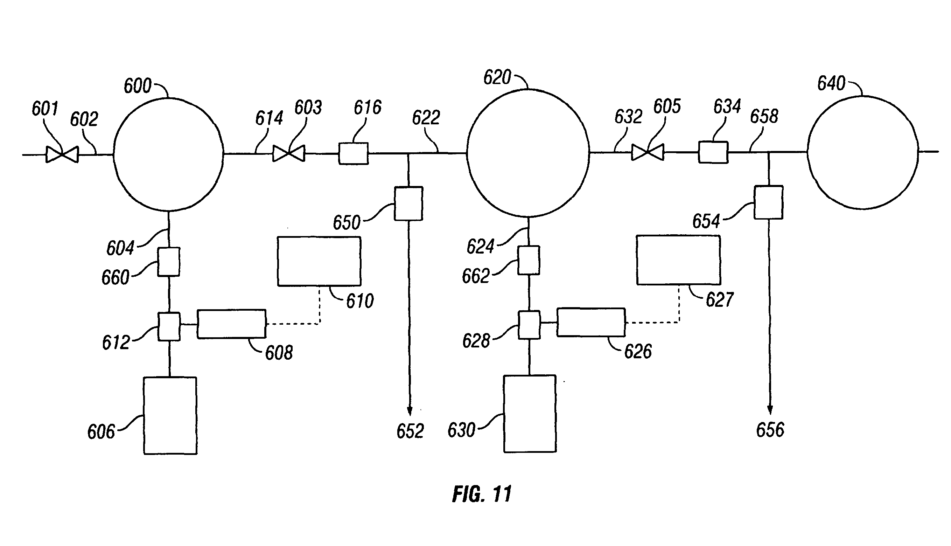

Referring to Figures 11 to 13 fluid flow paths of the invention for the preparation of dialysate on the disposable cartridge are shown schematically. It will be appreciated that all these flow paths are directly substitutable onto the cartridge as described with reference to Figures 4 to 6 to replace the dialysate preparation flow path described with reference thereto. It will also be appreciated that the method and apparatus according to the invention as described below does not need all of the features of the prior art as described above and is defined only by the scope of the claims. For example, it is anticipated that the air traps in the blood lines may be omitted. The below fluid flow paths for the preparation of dialysate reduce the complexity of the cartridge described above by removing the necessity for multiple reservoirs, each having level sensing, and therefore also greatly reducing the complexity of the control system required to prepare the dialysate.

Referring now to Figure 11 a flow path is shown for the preparation of dialysate prior to the flow balancer (Figure 4, 150). Sterile water enters the flow path into a first volumetric chamber comprising a first cavity 600 enclosed on one side by a membrane. The first cavity 600 has two inlets 602, 604, the first inlet 602 being for the supply of the sterile water and the second inlet 604 being for the supply of bicarbonate solution from a bicarbonate supply 606. Between the bicarbonate supply 606 and the inlet 604 is a bicarbonate pump 608 controlled by a controller 610 to draw bicarbonate solution from the bicarbonate reservoir 606 and supply it to the cavity first 600. The bicarbonate is drawn into the bicarbonate pump 608 and expelled from the bicarbonate pump 606 via dual check valve 612 to ensure bicarbonate solution always travels in the prescribed direction. The check valve 612 has integrated anti siphon means to prevent bicarbonate solution from siphoning into the cavity 606. The cavity 606 has an outlet 614 leading to an optional conductivity sensor 616 which by measuring conductivity obtains a signal indicative of the concentration of the bicarbonate in the water, as is common with conductivity sensors, the temperature may also be sensed to enhance the accuracy of the conductive sensor. In use, valve 601 is opened and valve 603 is closed and, either a negative pressure is applied to the membrane to draw it away from the first cavity 600 to draw water into the cavity via the inlet 602, or the water is supplied under pressure and the pressure of the water forces it into the cavity 600 thereby moving the membrane away from the first cavity 600. In either case the membrane is moved to fill the cavity 600 by means of a pressure differential across it. Associated with each stroke of the membrane, either during the drawing in of water, immediately proceeding it, or overlapping with the drawing in of water, a volume of bicarbonate solution corresponding to the volume required to achieve, in combination with the known volume of the first cavity 600, the required concentration of diluted bicarbonate solution, is pumped into the first cavity 600 by bicarbonate pump 608. Once the first cavity 600 is full, valve 601 is closed, valve 603 is open and, with valve 605 in a closed position, pressure is applied to the membrane to pump the mixture from the first cavity 600. The mixture of water and bicarbonate become thoroughly mixed by means of fluid turbulence within the cavity.

Downstream of the first chamber having a first cavity 600 is a second chamber having a second cavity 620 having an associated fluid inlet 622 and an acid inlet 624, and into which the mixture from the first cavity 600 flows when it is pumped therefrom. The fluid inlet 622 is connected to the outlet 614 of the first cavity 600 and the acid inlet 622 is connected, via an acid pump 626, controlled by controller 627, and associated anti-siphon check valve 628 to a supply of acid 630. The cavity 620 and associated acid pump 626 are arranged and operate substantially in the same way as the first cavity 600 to add a volumetric amount of acid into the second cavity 620 to mix therein with a volume of the dialysate entering the second cavity from the fluid inlet 622 as a membrane covering the cavity is exposed to a pressure differential across it (either by application of a negative pressure to draw it away from the cavity 620 or by the pressure of the fluid being pumped into it by means of pressure applied to the membrane of the first cavity 600), thereby achieving a predetermined ratiometric mixture of bicarbonate, acid and water, forming dialysate solution. Once the second cavity 620 is full, valve 603 is closed, valve 605 is opened, and a positive pressure is applied to the membrane to force it back towards and into the cavity pumping the dialysate solution from cavity 620 out of dialysate outlet 632. Downstream of the dialysate outlet 632 is an optional conductivity sensor 634 which creates a signal indicative of the concentration of the acid and bicarbonate mix in the dialysate solution. This signal may be used for monitoring the concentration of the dialysate.

The dialysate exiting the cavity 620 enters the first cavity 640 of the flow balancer which operates as described with reference to Figure 4 above.

As the amount of acid and bicarbonate added to the water to create dialysate varies from patient to patient according to their particular condition, then it is impossible to have one common cartridge that has exactly balanced sizes of first cavity, second cavity and flow balancer cavity, each to always accept exactly the full amount of fluid emitting from the previous cavity. Cavity 600, as the first in the series will always output a common volume. Cavity 620 will have at least some of this volume and add to it a variable amount of acid. The flow balancer will then accept the dialysate from the second cavity 620. As the second cavity 620 is fixed in maximum volume and has added into it a variable amount of acid, it will need to accept a variable amount of mixed fluid from the first cavity 600 to add to the volume of acid to make up the total volume of the second cavity 620. As a result there is a variable excess amount of the mixed fluid which the second cavity 620 can not accept. A sprung check valve 650 connects a fluid path between the first cavity 600 and second cavity 600 to a drain 652. The check valve is operable such that once the second cavity 620 is full, fluid pressure generated by the continued application of pressure to the membrane of the first cavity 600 is sufficient to open the check valve and allow the remaining fluid within the first cavity 600 to pass the valve and flow to a drain, thereby removing the excess mixed fluid from the system. By fully emptying the first cavity 600 every time it can be certain that the volumetric amount of bicarbonate solution added to the first cavity always mixes with the correct amount of water to produce the correct ratiometric mixture. Advantageously an optional second check valve 654 and associated second drain 656 may be placed in communication with a fluid conduit 658 leading from the second cavity 620 to the first cavity of the flow balancer 640. As it is hard to exactly match the volumes of the cavities due to manufacturing tolerances then by using this second valve 654 and drain 656 and by ensuring that the cavity 640 of the flow balancer is marginally smaller than the second cavity 620, it can be ensured that at all times during operation the flow balancer cavity 640 fully fills, and the second cavity 620 fully empties resulting in constant dilution ratios of the dialysate, and a known full cavity 640 of the flow balancer, thereby ensuring its accuracy. Conductivity sensors 660, 662 are optionally provided between the supplies of bicarbonate and acid which act to verify the concentration of the supplies. The signal from the sensors 660, 662 are fed back to the controllers 610, 627 such that the pumps can be altered to take into account any variation of the concentration of the acid or bicarbonate supplies 606, 636. These sensors are also useful in detecting if the supply of bicarbonate or acid run out and the process can be halted accordingly. The second cavity 620 is slightly larger in volume than the first cavity 600 (for example by the minimum acid requirement) such that the volume of mixed fluid that is drained is minimised.

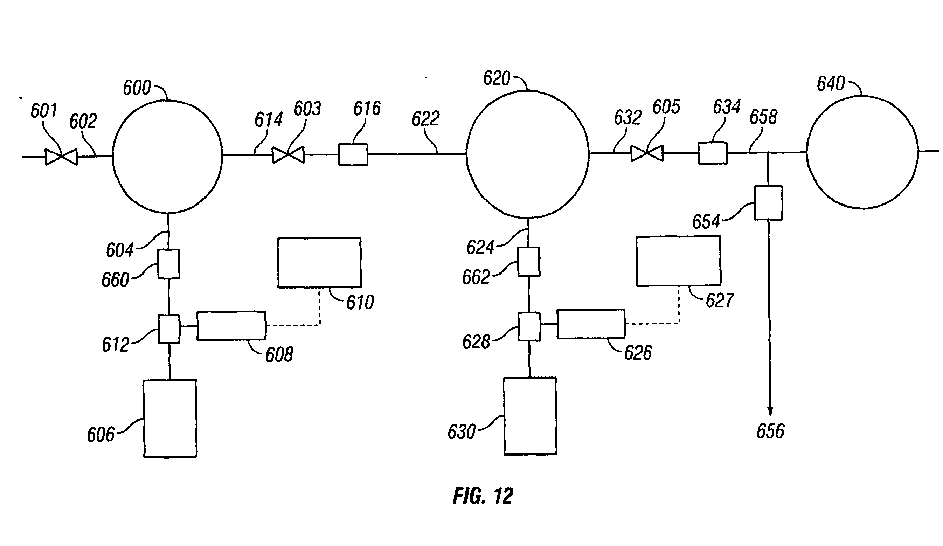

Referring to Figure 12 an alternative arrangement is shown which has only one check valve 654 between the second cavity 620 and the balancing pump cavity 640. In this arrangement the second cavity 620 is larger than the first cavity 600 such that the total contents of the first cavity 600, and the acid from the acid pump 626 can be accommodated within the second cavity 620. It will be appreciated that in this arrangement the second cavity 620 may not fully fill. When emptied by the application of pressure to the membrane the contents of the second cavity 620 is moved towards the flow balancer cavity 640, which is smaller in volume than the second cavity 620. Once the balancing pump cavity 640 is full, and excess fluid within the second cavity 640 is expelled from the fluid circuit via check valve 654 and flows to drain 656.

In the fluid circuits described with reference to Figures 11 and 12 the check valves between the fluid flow path and the drain open only under pressure to allow a one way flow of fluid from the fluid flow path and, when not open the check valve closes this flow path. The check valves 650,654 help to prevent any bacteria entering the fluid flow path.

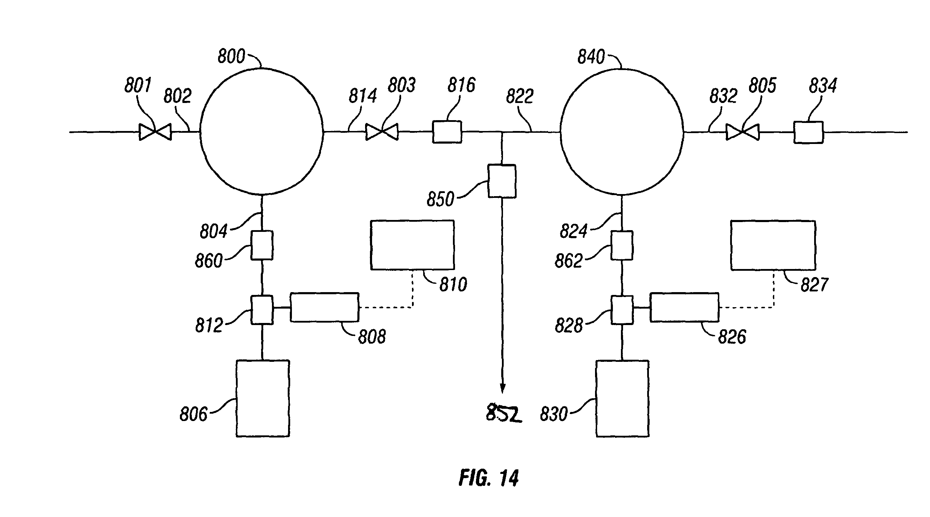

Referring to Figure 14 the system of figure 11 is shown wherein the chamber of the second mixing pump (620, Figure 11) and the chamber of the flow balancing pump (640, Figure 11) form one and the same common chamber 840, removing the need for a separate flow balancing chamber, such that the second dialysate solution base is added directly into the chamber 840 of the flow balancing pump.

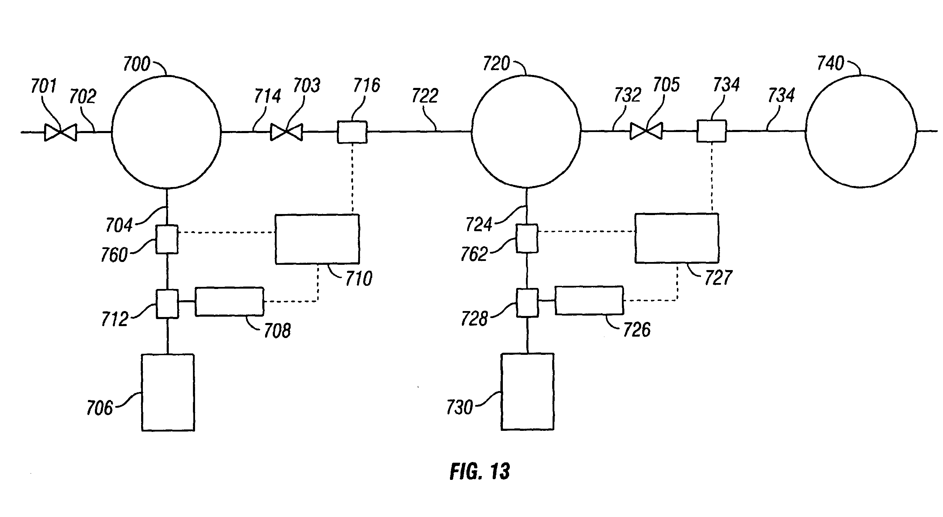

Referring to Figure 13 a flow path is shown for the preparation of dialysate prior to the flow balancer (Figure 4, 150). Sterile water enters the flow path into a second volumetric mixing chamber comprising a bicarbonate cavity 700 enclosed on one side by a membrane. The bicarbonate cavity 700 has two inlets 702, 704, the first inlet 702 being for the supply of the sterile water and the second inlet 704 being for the supply of bicarbonate solution from a bicarbonate supply 706. Between the bicarbonate supply 706 and the inlet 704 is a bicarbonate pump 708 controlled by a controller 710 to draw bicarbonate solution from the bicarbonate reservoir 706 and supply it to the bicarbonate cavity 700. The bicarbonate is drawn into the bicarbonate pump 708 and expelled from the bicarbonate pump 706 via dual check valve 712 to ensure bicarbonate solution always travels in the prescribed direction. The check valve 712 has integrated anti siphon means to prevent bicarbonate solution from siphoning into the cavity 706. The cavity 700 has an outlet 714 leading to a conductivity sensor 716 which by measuring conductivity obtains a signal indicative of the concentration of the bicarbonate in the water. In use, valve 701 is opened and valve 703 is closed and, either a negative pressure is applied to the membrane to draw it away from the bicarbonate cavity 700 to draw water into the cavity via the inlet 702, or the water is supplied under pressure and the pressure of the water forces it into the cavity 700 thereby moving the membrane away from the bicarbonate cavity 700. In either case the membrane is moved to fill the cavity 700 by means of a pressure differential across it. Associated with each stroke of the membrane, either during the drawing in of water, immediately proceeding it, or overlapping with the drawing in of water, a volume of bicarbonate solution corresponding to the volume required to achieve, in combination with the known volume of the bicarbonate cavity 700, the required concentration of diluted bicarbonate solution, is pumped into the bicarbonate cavity 700 by bicarbonate pump 708. Once the bicarbonate cavity 700 is full, valve 701 is closed, valve 703 is open and, with valve 705 in a closed position, pressure is applied to the membrane to pump the mixture from the bicarbonate cavity 700. The mixture of water and bicarbonate becomes thoroughly mixed by means of fluid turbulence within the cavity. Optionally, the signal from the conductivity sensor 716 can be input to the controller 710 which can then adjust the pump 708 to increase or decrease the amount pumped on each stroke of the membrane to achieve the desired bicarbonate concentration in the liquid emitting from the cavity 700. Although there may be benefits of this feedback, it is not required to make the system work as equilibrium will soon be reached based on the known volume of bicarbonate added from the bicarbonate pump 708. When not used for feedback control the conductivity sensor monitors the concentration and can raise an alarm or stop the process if the concentration goes out of specific tolerances. Downstream of the second mixing pump chamber having a bicarbonate cavity 700 is a first mixing pump chamber having an acid cavity 720 having an associated fluid inlet 722 and an acid inlet 724, and into which the mixture from the bicarbonate cavity 700 flows when it is pumped therefrom. The fluid inlet 722 is connected to the outlet 714 of the bicarbonate cavity 700 and the acid inlet 724 is connected, via an acid pump 726, controlled by controller 727, and associated anti-siphon check valve 728 to a supply of acid 730. The cavity 720 and associated acid pump 726 are arranged and operate substantially in the same way as the bicarbonate cavity 700 to add a volumetric amount of acid into the acid cavity 720 to mix therein with a volume of the dialysate entering the acid cavity from the fluid inlet 722 as a membrane covering the cavity is exposed to a pressure differential across it (either by application of a negative pressure to draw it away from the cavity 720 or by the pressure of the fluid being pumped into it by means of pressure applied to the membrane of the bicarbonate cavity 700). Once the acid cavity 720 is full, valve 703 is closed, valve 705 is opened, and a positive pressure is applied to the membrane to force it back towards and into the cavity pumping the dialysate solution from cavity 720 out of dialysate outlet 732. Downstream of the dialysate outlet 732 is a conductivity sensor 734 which creates a signal indicative of the concentration of the acid and bicarbonate mix in the dialysate solution. Optionally, the signal can be fed back to the controller 727 to vary the amount of acid pumped by acid pump 726 to achieve a predetermined ratiometric mixture of bicarbonate, acid and water, forming dialysate solution; this enables more dynamic control of the system and facilitates changing of concentration partway through a treatment process. When not used for feedback control the conductivity sensor 734 monitors the concentration and can raise an alarm or stop the process if the concentration goes out of specific tolerances. The dialysate exiting the cavity 720 enters the flow balancer cavity 740 of the flow balancer which operates as described with reference to Figure 4 above.

In operation as a varying amount of bicarbonate and acid solution may be added in the bicarbonate 700 and acid 720 cavities respectively, the volumes varying depending on patient specific requirements, it is not possible to balance a system such that the volume of the cavities exactly matches the volumes of fluid pumped on each stroke, as these cumulative increase in flow from chamber to chamber due to differing amounts of bicarbonate and acid added will change from patient to patient. Therefore when the cartridge with the dialysate mixing flow path as described in reference to Figure 13 is used the method of pumping will result in the bicarbonate and acid cavities never fully emptying. Once the system has been set up and run for a brief time to stabilise the residual volumes in the cavities, it will produce a constant ratiometric mixture of dialysate solution. However it is important, when used with the method of flow balancing described herein, that the flow balancer cavity 740 is always completely filled. For that reason it is preferable that the acid pump cavity 720 has a minimum toleranced volume greater than the flow balancer cavity 740, and the bicarbonate cavity 700 has a minimum toleranced volume greater than the maximum toleranced volume of the acid cavity 700. In this way, as fluid passes from one pump to the next going downstream, the cavities are always completely filled. As an alternative to this arrangement, providing that the bicarbonate cavity 720 has a minimum toleranced volume greater than the flow balancer cavity 740, the acid cavity 720 can be any size providing that it has a minimum toleranced volume greater that that flow balancer cavity, i.e. it can be greater in volume than the bicarbonate cavity. Sensors 760 and 762 may be used as safety alarms, for example they may be configured to alarm if the bicarbonate or acid runs out and the pump starts to push air into the system. In this way the system can be stopped as soon as any air is detected.

Referring to 15 the system of Figure 13 is shown wherein the balancing pump 940 is configured to receive a mixture of water and first dialysate base solution from the first mixing pump and a second dialysate solution base solution. In use, sterile water enters the flow path into a first volumetric chamber comprising a first cavity 900 enclosed on one side by a membrane. The first cavity 900 has two inlets 902, 904, the first inlet 902 being for the supply of the sterile water and the second inlet 904 being for the supply of bicarbonate solution from a bicarbonate supply 906. Between the bicarbonate supply 906 and the inlet 904 is a bicarbonate pump 908 controlled by a controller 910 to draw bicarbonate solution from the bicarbonate reservoir 906 and supply it to the cavity first 900. The cavity 900 has an outlet 914 leading to a conductivity sensor 916 which by measuring conductivity obtains a signal indicative of the concentration of the bicarbonate in the water. In use, pump operates as described with reference to Figure 13. Downstream of the first chamber having a first cavity 900 is a balancing chamber having a balancing cavity 940 having an associated fluid inlet 922 and an acid inlet 924, and into which the mixture from the first cavity 900 flows when it is pumped therefrom. The fluid inlet 922 is connected to the outlet 914 of the first cavity 900 and the acid inlet 924 is connected, via an acid pump 926, controlled by controller 927, and associated anti-siphon check valve 928 to a supply of acid 930. The balancing cavity 940 and associated acid pump 926 are arranged and operate substantially in the same way as the first cavity 900 to add a volumetric amount of acid into the balancing cavity 940 to mix therein with a volume of the dialysate entering the mixing cavity from the fluid inlet 922 as a membrane covering the balancing cavity 940 is exposed to a pressure differential across it (either by application of a negative pressure to draw it away from the balancing cavity 940 or by the pressure of the fluid being pumped into it by means of pressure applied to the membrane of the first cavity 900). Once the balancing cavity 940 is full, valve 903 is closed, valve 905 is opened, and a positive pressure is applied to the membrane to force it back towards and into the cavity pumping the dialysate solution from balancing cavity 940 out of dialysate outlet 932. Downstream of the dialysate outlet 932 is a conductivity sensor 934 which creates a signal indicative of the concentration of the acid and bicarbonate mix in the dialysate solution. Optionally, the signal can be fed back to the controller 927 to vary the amount of acid pumped by acid pump 926 to achieve a predetermined ratiometric mixture of bicarbonate, acid and water, forming dialysate solution, this enables more dynamic control of the system and facilitates changing of concentration partway through a treatment process. When not used for feedback control the conductivity sensor 934 can monitor the concentration and can raise an alarm or stop the process if the concentration goes out of specific tolerances. The balancing cavity 940 operates in a flow balancing mode described with reference to Figure 4 above.

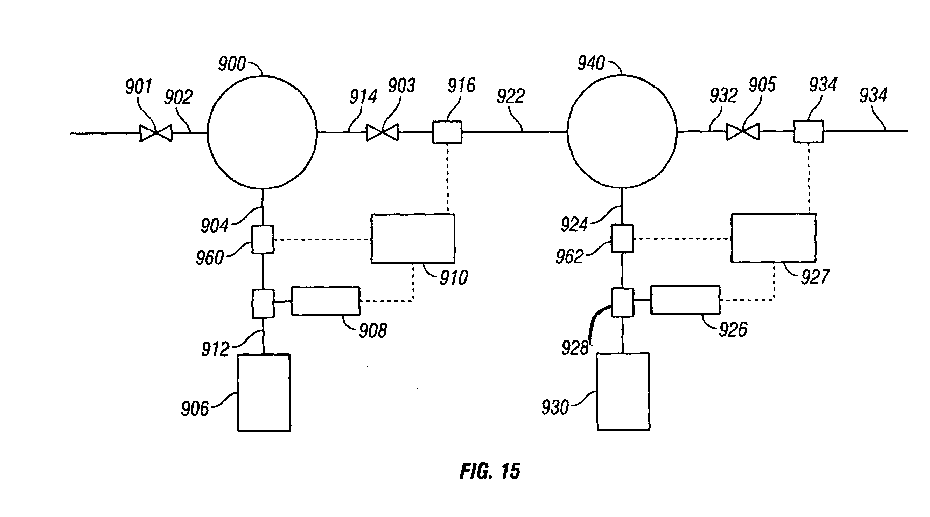

Similarly to the dialysate mixing path of Figure 13, in operation, as a varying amount of bicarbonate and acid solution may be added in the first mixing cavity 900 and balancing cavity 940 respectively, it is not possible to balance a system such that the volume of the cavities exactly matches the volumes of fluid pumped on each stroke, as these cumulative increase in flow from chamber to chamber due to differing amounts of bicarbonate and acid added will change from patient to patient. It is important that the flow balancer cavity 940 is always completely filled. For that reason it is preferable that the first cavity 900 has a minimum toleranced volume greater than the flow balancer cavity 940. Therefore when the cartridge with the dialysate mixing flow path as described in reference to Figure 15 is used the method of pumping will result in the first cavity never fully emptying. Once the system has been set up and run for a brief time to stabilise it will produce a constant ratiometric mixture of dialysate solution. Sensors 960 and 962 may be used as safety alarms, for example they may be configured to alarm if the bicarbonate or acid runs out and the pump starts to push air into the system. In this way the system can be stopped as soon as any air is detected.

It will be appreciated that the above description is given by way of example only and it is anticipated that various changes may be made to the specific arrangement of components without departing from the scope of the invention as defined by the claims, for example check valves 612, 628 could easily be substituted with on/off inlet and outlet valves, and sensors 616,634,660,662 when used for monitoring purposes may be included or omitted. It will further be appreciated that the bicarbonate and acid need not be directly introduced into the pump cavities and may instead be introduced into the inlet conduits upstream of the pump cavities.

## Claims

1. A cartridge (10) for use in a hemodialysis machine (1), the cartridge comprising:
a dialysate flow path including a dialyser (80), the dialysate flow path for delivering a flow of dialysate to the dialyser (80);
a first mixing pump (600) comprising a chamber having a fixed volume between a concave recess and a flexible membrane (22), said chamber for receiving a predetermined volume of a first dialysate solution base, and a volume of water;
a flow balancing pump (640) comprising: a chamber having a fixed volume between a concave recess and a flexible membrane (22), and inlet through which it receives dialysate; a fluid flow path connecting the first mixing pump (600) to the flow balancing pump (640);
the cartridge (10) further comprising:
a first check valve (650, 654) having an inlet in fluid communication with said fluid flow path, said check valve (650, 654) configured to open if the pressure in the fluid flow path exceeds a predetermined pressure to allow excess fluid in the fluid flow path to flow through the first check valve (650, 654) to a drain (652), and to close when the pressure in the fluid flow path falls back below said predetermined pressure.

2. A cartridge (10) according to claim 1 further comprising:
a second mixing pump (620) downstream of the first mixing pump (600), the second mixing pump (620) comprising a chamber having a fixed volume between a concave recess and a flexible membrane (22) for receiving a predetermined volume of a second dialysate solution base and the mixture of water and first dialysate solution base from the first mixing pump (620), and wherein
the inlet of the first check valve (650) is in fluid communication with the fluid flow path between said first and second mixing pumps.

3. A cartridge (10) according to claim 2 further comprising:
a second check valve (654) having an inlet, said second check valve inlet in fluid communication with the fluid flow path between the second mixing pump (620) and the balancing pump (640), wherein
the second check valve (654) is configured to open if the pressure in the fluid flow path between the second mixing pump (620) and the balancing pump (640) increases above a predetermined pressure to allow excess fluid in the fluid flow path to flow through the second check valve (654) to a drain (656), and to close when the pressure in the fluid flow path falls back below the said predetermined pressure.

4. A cartridge (10) according to claim 2 wherein the chamber of the second mixing pump (620) and the chamber of the flow balancing pump (640) are one and the same chamber.

5. A cartridge (10) according to claim 1 further comprising:
a second mixing pump (620) downstream of the first mixing pump (600), the second mixing pump (620) comprising a chamber having a fixed volume between a concave recess and a flexible membrane (22), said chamber for receiving a predetermined volume of a second dialysate solution base and the mixture of water and first dialysate solution base from the first mixing pump (600), and wherein
the inlet of the first check valve (654) is in fluid communication with the fluid flow path between the second mixing pump (620) and the flow balancing pump (640).

6. A cartridge (10) according to claim 5 wherein the second mixing pump (620) has a volume greater than the first mixing pump (600).

7. A cartridge (10) according to claim 1 further comprising:
a first dialysate base pump (608), said first dialysate base pump (608) configured to add a volume of the first dialysate base to the first mixing pump (600), and
a controller (610) to control the first dialysate base pump (608).

8. A cartridge (10) according to claim 7 further comprising:
a first sensor (616) in the fluid flow path, said sensor (616) downstream of the first mixing pump (600), the sensor (616) arranged to feedback a signal indicative of the concentration of the mixture of first dialysate solution base and water exiting the first mixing pump (600), to the controller (610), and wherein
the controller (610) is configured to control the first dialysate solution base pump (608) to achieve a predetermined ratiometric mix of first dialysate solution base and water.

9. A cartridge (10) according to claim 7 or claim 8 further comprising:
a first dialysate solution base sensor (660) that creates a signal indicative of the concentration of the first dialysate solution base, and wherein
the controller (610) receives said signal and uses it to control the first dialysate solution base pump (608) to achieve a predetermined ratiometric mix of first dialysate solution base and water in the chamber of the first mixing pump (600).

10. A cartridge (10) according to claim 2 further comprising:
a second dialysate base pump (626) configured to add a volume of the second dialysate base to the second mixing pump (620), and
a controller (627) to control the second dialysate base pump (626).

11. A cartridge (10) according to claim 10 further comprising:
a sensor (634) downstream of the second mixing pump (620) that creates a signal indicative of the concentration of the mixture of second dialysate solution base and the mixture of water and first dialysate solution base exiting the second mixing pump (620), and wherein
the controller (627) receives said signal and uses it to control the second dialysate solution base pump (620) to achieve a predetermined ratiometric mix of second dialysate solution base with the mixture of water and first dialysate solution.

12. A cartridge (10) according to claim 10 or claim 11 further comprising:
a second dialysate solution base sensor that creates a signal indicative of the concentration of the second dialysate solution base, and wherein
the controller (627) receives said signal and uses it to control the second dialysate solution base pump (620) to achieve a predetermined ratiometric mix of second dialysate solution base with the mixture of water and first dialysate solution in the chamber of the second mixing pump (620).

13. A cartridge (10) for use in a hemodialysis machine (1), the cartridge (10) comprising:
a dialysate flow path including a dialyser, the dialysate flow path for delivering a flow of dialysate to the dialyser;
a first mixing pump (720; 800; 900) comprising a chamber having a fixed volume between a recess and a flexible membrane (22), said chamber for receiving a predetermined volume of a first dialysate solution base, and a volume of water;
a first dialysate solution base pump (708; 808; 908), controlled by a controller (710; 810; 910), for adding a predetermined volume of a first dialysate solution base to the first mixing pump chamber (720; 800; 900);
a flow balancing pump (740; 840; 940) comprising a chamber having a fixed volume between a recess and a flexible membrane (22) and an inlet through which it receives dialysate; and
a fluid flow path connecting the first mixing pump (720; 800; 900) to the flow balancing pump (740; 840; 940);
wherein the minimum toleranced volume of the first mixing pump (720; 800; 900) is equal to, or greater than, the maximum toleranced volume of the balancing pump (740; 840; 940).

14. A cartridge (10) according to claim 13 further comprising:
a second dialysate mixing pump (700) upstream of the first dialysate mixing pump (720), the second mixing pump (700) comprising a chamber having a fixed volume between a recess and a flexible membrane (22), said chamber for receiving a predetermined volume of a second dialysate solution base, and a volume of water;
a second dialysate solution base pump (726), controlled by a controller (727), for adding a predetermined volume of a second dialysate solution base to the second mixing pump chamber (700); and
a second fluid flow path connecting the second (700) and the first (720) mixing pumps for delivering a mixture of water and second dialysate base solution to the chamber of the first mixing pump (720).

15. A cartridge (10) according to claim 14 wherein;
the minimum toleranced volume of the second mixing pump (700) is equal to, or greater than, the maximum toleranced volume of the first mixing pump (720).

16. A cartridge (10) according to claim 13 or claim 14 wherein the cartridge (10) further comprises:
a sensor (734) downstream of the first mixing pump (720), said sensor (734) configured to create a signal indicative of the concentration of the fluid exiting the first mixing pump (720).

17. A cartridge (10) according to claim 14 wherein the cartridge (10) further comprises:
a sensor (716) downstream of the second mixing pumps (700), said sensor (716) configured to create a signal indicative of the concentration of the fluid exiting the second mixing pumps (700).

18. A cartridge (10) according to claim 13 wherein:
the balancing pump (840; 940) is configured to receive into its chamber: a mixture of water and first dialysate base solution from the chamber of the first mixing pump (800; 900), and a second dialysate solution base solution from a second dialysate solution base pump (826; 926).

19. A cartridge (10) according to claim 18 wherein the cartridge further comprises:
a sensor (816; 916) downstream of the balancing pump (800; 900), said sensor (816; 916) configured to create a signal indicative of the concentration of the fluid exiting the chamber of the balancing pump (800; 900).

20. A cartridge (10) according to claim 16, claim 17 or claim 19 wherein
in use, feedback signals from the sensors (734; 716; 916) are fed to respective controllers (710; 727; 910) of the first and second dialysate solution base pumps, the respective controllers configured to control the first (708; 908) and second (726; 926) dialysate solution base pumps to modify the amount of first and second dialysate solution base they add to the first and second mixing pumps (720; 700), or first mixing pump (900) and balancing pump (740; 940), respectively, so as to achieve a predetermined ratiometric mixture of first and second dialysate solution base and water.

21. A cartridge (10) according to claim 16, claim 17 or claim 19 wherein
in use, signals from the sensors (734; 716; 916) are monitored and cause an alarm if the signal generated falls outside of specific parameters.

22. A method of mixing a dialysate solution in the cartridge (10) according to any one of claims 13 to 21, the method comprising:
filling the chamber of the first mixing pump (720; 800; 900) with a predetermined volume of first dialysate base solution and water;
pumping a proportion of the mixture in the chamber of the first mixing pump (720; 800; 900) into the chamber of the balancing pump (740; 840; 940); and
retaining a proportion of the mixture of first dialysate base solution and water in the chamber of the first mixing pump (720; 800; 900).

23. The method according to claim 22 wherein the method further comprises:
measuring a property of the dialysate base solution and water exiting the first mixing chamber (720; 800; 900) to generate a signal indicative of its concentration,
determining if the concentration is greater or lesser than the required concentration;
controlling the first dialysate solution base pump (708; 808; 908) to increase or decrease the amount of first dialysate solution base added to the chamber of the first mixing pump (720; 800; 900) accordingly, and repeating the method until the required concentration is achieved.

24. The method according to claim 22 or claim 23 wherein the method further comprises:
filling the chamber of the second mixing pump (700) with a predetermined volume of second dialysate base solution and water;
pumping a proportion of the mixture of second dialysate base solution and water, and a predetermined volume of first dialysate solution base, into the chamber of the first mixing pump (720);
retaining a proportion of the mixture of second dialysate base solution and water in the chamber of the second mixing pump (700).

25. The method according to claim 24 wherein the method further comprises:
measuring a property of the second dialysate base solution and water exiting the second mixing chamber to generate a signal indicative of its concentration,
determining if the concentration is greater or lesser than the required concentration;
controlling the second dialysate solution base pump (726) to increase or decrease the amount of second dialysate solution base added to the chamber of the second mixing pump (700) accordingly.

26. The method according to any one of claims 22 to 25 wherein the method comprises:
repeating the steps of filling and partially emptying the chambers until equilibrium is achieved and the required dialysate concentrations is achieved.

27. The cartridge (10) according to any one of claims 1 to 21 wherein the cartridge (10) further comprises a blood flow path for carrying a volume of blood to be treated in the dialyser, or wherein the cartridge (10) is disposable.

## Patentansprüche

1. Einsatz (10) zum Gebrauch in einer Hämodialyse-Anlage (1) umfassend:
eine einen Dialysator (80) einschließende Dialysat-Flussbahn, wobei die Dialysat-Flussbahn zur Abgabe eines Flusses von Dialysat zum Dialysator (80) dient;
eine erste Mischpumpe (600), umfassend eine Kammer, welche ein festes Volumen zwischen einer konkaven Mulde und einer flexiblen Membran (22) aufweist, wobei die Kammer zur Aufnahme eines vorbestimmten Volumens einer ersten Dialysatlösung-Basis und eines Volumens von Wasser dient;
eine Flussausgleichpumpe (640) umfassend: eine Kammer, welche ein festes Volumen zwischen einer konkaven Mulde und einer flexiblen Membran (22) und einen Einlass, durch den sie Dialysat empfängt, aufweist, eine Fluid-Flussbahn, welche die erste Mischpumpe (600) mit der Flussausgleichpumpe (640) verbindet;
wobei der Einsatz (10) des Weiteren umfasst:
ein erstes Prüfventil (650, 654) mit einem Einlass in Fluidkommunikation mit der Fluid-Flussbahn, wobei das Prüfventil (650, 654) eingerichtet ist,
zu öffnen, wenn der Druck in der Fluid-Flussbahn einen vorbestimmten Druck überschreitet, um überschüssigem Fluid in der Fluid-Flussbahn zu erlauben, durch das erste Prüfventil (650, 654) zu einem Ablass (652) zu fließen, und
zu schließen, wenn der Druck in der Fluid-Flussbahn unter den vorbestimmten Druck zurückfällt.

2. Einsatz (10) nach Anspruch 1, des Weiteren umfassend:
eine zweite Mischpumpe (620) in Flussrichtung hinter der ersten Mischpumpe (600), wobei die zweite Mischpumpe (620) eine Kammer umfasst, welche ein festes Volumen zwischen einer konkaven Mulde und einer flexiblen Membran (22) zur Aufnahme eines vorbestimmten Volumens einer zweiten Dialysatlösung-Basis und der Mischung aus Wasser und erster Dialysatlösung-Basis von der ersten Mischpumpe (620) aufweist, und wobei
der Einlass des ersten Prüfventils (650) in Fluidkommunikation mit der Fluid-Flussbahn zwischen der ersten und zweiten Mischpumpe ist.

3. Einsatz (10) nach Anspruch 2, des Weiteren umfassend:
ein zweites Prüfventil (654) mit einem Einlass, wobei der zweite Prüfventileinlass in Fluidkommunikation mit der Fluid-Flussbahn zwischen der zweiten Mischpumpe (620) und der Ausgleichpumpe (640) ist, wobei
das zweite Prüfventil (654) eingerichtet ist,
zu öffnen, wenn der Druck in der Fluid-Flussbahn zwischen der zweiten Mischpumpe (620) und der Ausgleichpumpe (640) über einen vorbestimmten Druck steigt, um überschüssigem Fluid in der Fluid-Flussbahn zu erlauben, durch das zweite Prüfventil (654) zu einem Ablass (656) zu fließen, und
zu schließen, wenn der Druck in der Fluid-Flussbahn unter den vorbestimmten Druck zurückfällt.

4. Einsatz (10) nach Anspruch 2, wobei die Kammer der zweiten Mischpumpe (620) und die Kammer der Flussausgleichpumpe (640) ein und dieselbe Kammer sind.

5. Einsatz (10) nach Anspruch 1, des Weiteren umfassend:
eine zweite Mischpumpe (620) in Flussrichtung hinter der ersten Mischpumpe (600), wobei die zweite Mischpumpe (620) eine Kammer umfasst, welche ein festes Volumen zwischen einer konkaven Mulde und einer flexiblen Membran (22) aufweist, wobei die Kammer zur Aufnahme eines vorbestimmten Volumens einer zweiten Dialysatlösung-Basis und der Mischung von Wasser und erster Dialysatlösung-Basis von der ersten Mischpumpe (600) dient, und wobei
der Einlass des ersten Prüfventils (754) in Fluidkommunikation mit der Fluid-Flussbahn zwischen der zweiten Mischpumpe (620) und der Flussausgleichpumpe (640) ist.

6. Einsatz (10) nach Anspruch 5, wobei die zweite Mischpumpe (620) ein größeres Volumen aufweist als die erste Mischpumpe (600).

7. Einsatz (10) nach Anspruch 1, des Weiteren umfassend:
eine erste Dialysatbasis-Pumpe (608), wobei die erste Dialysatbasis-Pumpe (608) eingerichtet ist, ein Volumen der ersten Dialysatbasis zu der ersten Mischpumpe (600) hinzuzufügen und
eine Steuerung (610), um die erste Dialysatbasis-Pumpe (608) zu steuern.

8. Einsatz (10) nach Anspruch 7, des Weiteren umfassend:
einen ersten Sensor (616) in der Fluid-Flussbahn, wobei der Sensor (616) flussabwärts von der ersten Mischpumpe (600) ist, wobei der Sensor (616) eingerichtet ist, ein Signal zur Steuerung (610) zurückzukoppeln, welches die Konzentration der aus der ersten Mischpumpe (600) austretenden Mischung der ersten Dialysatlösung-Basis und Wasser anzeigt und wobei
die Steuerung (610) eingerichtet ist, die erste Dialysatlösung-Basis-Pumpe (608) zu steuern, um eine vorbestimmte ratiometrische Mischung der ersten Dialysatlösung-Basis und Wasser zu erreichen.

9. Einsatz (10) nach Anspruch 7 oder Anspruch 8, des Weiteren umfassend:
einen ersten Dialysatlösung-Basis-Sensor (660), welches ein die Konstellation der ersten Dialysatlösung-Basis anzeigendes Signal erzeugt, und wobei
die Steuerung (610) dieses Signal empfängt und es nutzt, die erste Dialysatlösung-Basis-Pumpe (608) zu steuern, um eine vorbestimmte ratiometrische Mischung der ersten Dialysatlösung-Basis und Wasser in der Kammer der ersten Mischpumpe (600) zu erreichen.

10. Einsatz (10) nach Anspruch 2, des Weiteren umfassend:
eine zweite Dialysatbasis-Pumpe (626), welche eingerichtet ist, ein Volumen der zweiten Dialysatbasis zu der zweiten Mischpumpe (620) hinzuzufügen, und
eine Steuerung (627), um die zweite Dialysatbasis-Pumpe (626) zu steuern.

11. Einsatz (10) nach Anspruch 10, des Weiteren umfassend:
einen Sensor (634) flussabwärts zur zweiten Mischpumpe (620), welcher ein Signal erzeugt, welches die Konzentration der aus der zweiten Mischpumpe (620) austretenden Mischung der zweiten Dialysatlösung-Basis und der Mischung aus Wasser und erster Dialysatlösung-Basis anzeigt, und wobei
die Steuerung (627) das besagte Signal empfängt und es zur Kontrolle der zweiten Dialysatlösung-Basis-Pumpe (620) nutzt, um eine vorbestimmte ratiometrische Mischung der zweiten Dialysatlösung-Basis mit der Mischung aus Wasser und erster Dialysatlösung zu erreichen.

12. Einsatz (10) nach Anspruch 10 oder Anspruch 11, des Weiteren umfassend:
einen zweiten Dialysatlösung-Basis-Sensor, welcher ein die Konzentration der zweiten Dialysatlösung-Basis anzeigendes Signal erzeugt, und wobei
die Steuerung (627) das Signal empfängt und es benutzt, um die zweite Dialysatlösung-Basis-Pumpe (620) zu steuern, um eine vorbestimmte ratiometrische Mischung der zweiten Dialysatlösung-Basis mit der Mischung aus Wasser und erster Dialysatlösung in der Kammer der zweiten Mischpumpe (620) zu erreichen.

13. Einsatz (10) zur Nutzung in einer Hämodialyse-Anlage (1), umfassend:
eine einen Dialysator einschließende Dialysat-Flussbahn, wobei die Dialysat-Flussbahn zur Abgabe eines Dialysatflusses zum Dialysator dient;
eine erste Mischpumpe (720; 800; 900), umfassend eine Kammer mit einem festen Volumen zwischen einer Mulde und einer flexiblen Membran (22), wobei die Kammer zur Aufnahme eines vorbestimmten Volumens einer ersten Dialysatlösung-Basis und eines Volumens von Wasser dient;
eine erste Dialysatlösung-Basis-Pumpe (708; 808; 908), gesteuert durch eine Steuerung (710; 810; 910), zum Hinzufügen eines vorbestimmten Volumens einer ersten Dialysatlösung-Basis zu der ersten Mischpumpenkammer (720; 800; 900):
eine Flussausgleichpumpe (740; 840; 940), umfassend eine Kammer mit einem festen Volumen zwischen einer Mulde und einer flexiblen Membran (22) und einem Einlass, durch den es Dialysat empfängt; und
eine Fluid-Flussbahn, welche die erste Mischpumpe (720; 800; 900) mit der Flussausgleichpumpe (740; 840; 940) verbindet;
wobei das minimale tolerierte Volumen der ersten Mischpumpe (720; 800; 900) gleich ist zu dem oder größer ist als das maximale(n) tolerierte(n) Volumen der Ausgleichpumpe (740; 840; 940).

14. Einsatz (10) nach Anspruch 13, des Weiteren umfassen:
eine zweite Dialysat-Mischpumpe (700) stromaufwärts zur ersten Dialysat-Mischpumpe (720), wobei die zweite Mischpumpe (700) eine ein festes Volumen zwischen einer Mulde und einer flexiblen Membran (22) aufweisende Kammer umfasst, wobei die Kammer zur Aufnahme eines vorbestimmten Volumens einer zweiten Dialysatlösung-Basis dient, und ein Volumen von Wasser;
eine zweite Dialysatlösung-Basis-Pumpe (726), gesteuert durch die Steuerung (727), um ein vorbestimmtes Volumen einer zweiten Dialysatlösung-Basis zu der zweiten Mischpumpenkammer (700) hinzuzufügen, und
eine zweite Fluid-Flussbahn, welche die zweite (700) und die erste (720) Mischpumpen verbindet, um eine Mischung aus Wasser und zweiter Dialysat-Basislösung zu der Kammer der ersten Mischpumpe (720) zu liefern.

15. Einsatz (10) nach Anspruch 14, wobei das minimale tolerierte Volumen der zweiten Mischpumpe (700) gleich ist zu dem oder größer ist als das maximale(n) tolerierte(n) Volumen der ersten Mischpumpe (720).

16. Einsatz (10) nach Anspruch 13 oder Anspruch 14, wobei der Einsatz (10) des Weiteren umfasst:
einen Sensor (734) flussabwärts zur ersten Mischpumpe (720), wobei der Sensor (734) konfiguriert ist, ein Signal zu erzeugen, welches die Konzentration des die erste Mischpumpe (720) austretende Fluids anzeigt.

17. Einsatz (10) nach Anspruch 14, wobei der Einsatz (10) des Weiteren umfasst:
einen Sensor (716) stromabwärts zur zweiten Mischpumpe (700), wobei der Sensor (716) konfiguriert ist, ein Signal zu erzeugen, welches die Konzentration des aus der zweiten Mischpumpe (700) austretenden Fluids anzeigt.

18. Einsatz (10) nach Anspruch 13, wobei:
die Ausgleichpumpe (840; 940) konfiguriert ist, in seine Kammer aufzunehmen: eine Mischung aus Wasser und erster Dialysat-Basislösung von der Kammer der ersten Mischpumpe (800; 900) und eine zweite Dialysatlösung-Basislösung von einer zweiten Dialysatlösung-Basis-Pumpe (826; 926).

19. Einsatz (10) nach Anspruch 18, wobei der Einsatz des Weiteren umfasst:
einen Sensor (816; 916) stromabwärts der Ausgleichpumpe (800; 900), wobei der Sensor (816; 916) konfiguriert ist, ein die Konzentration des die Kammer der Ausgleichpumpe (800; 900) verlassenden Fluids anzeigendes Signal zu erzeugen.

20. Einsatz (10) nach Anspruch 16, Anspruch 17 oder Anspruch 19, wobei
im Gebrauch Rückkoppelsignale von den Sensoren (734; 716; 916) zu zugehörigen Steuerungen (710; 727; 910) der ersten und zweiten Dialysatlösung-Basis-Pumpen zugeführt werden, wobei die zugehörigen Steuerungen konfiguriert sind, die ersten (708; 908) und zweiten (726; 926) Dialysatlösung-Basis-Pumpen zu steuern, um die Menge der ersten und zweiten Dialysatlösung-Basis zu modifizieren, welche sie zu den ersten und zweiten Mischpumpen (720; 700) hinzufügen, beziehungsweise erste Mischpumpe (900) und Ausgleichpumpe (740; 940) zu steuern, so dass eine vorbestimmte ratiometrische Mischung von erster und zweiter Dialysatlösung-Basis und Wasser erreicht wird.

21. Einsatz (10) nach Anspruch 16, Anspruch 17 oder Anspruch 19, wobei
im Gebrauch, Signale der Sensoren (734; 716; 916) angezeigt werden und einen Alarm begründen, wenn das erzeugte Signal außerhalb spezifischer Parameter fällt.

22. Verfahren zum Mischen einer Dialysatlösung in dem Einsatz (10) gemäß einem der Ansprüche 13 bis 21, umfassend:
Füllen der Kammer der ersten Mischpumpe (720; 800; 900) mit einem vorbestimmten Volumen der ersten Dialysat-Basislösung und Wasser;
Pumpen eines Anteils der Mischung in der Kammer der ersten Mischpumpe (720; 800; 900) in die Kammer der Ausgleichpumpe (740; 840; 940) und
Einbehalten eines Anteils der Mischung von erster Dialysat-Basislösung und Wasser in der Kammer der ersten Mischpumpe (720; 800; 900).

23. Verfahren nach Anspruch 22, wobei das Verfahren des Weiteren umfasst:
Messen einer Eigenschaft der aus der ersten Mischkammer (720; 800; 900) austretenden Dialysat-Basislösung und Wasser, um ein deren Konzentration anzeigendes Signal zu generieren;
Bestimmen, ob die Konzentration größer oder geringer ist als die benötigte Konzentration;
Steuern der ersten Dialysatlösung-Basis-Pumpe (708; 808; 908), um die Menge der zur Kammer der ersten Mischpumpe (720; 800; 900) hinzugefügten ersten Dialysatlösung-Basis entsprechend zu erhöhen oder zu erniedrigen, und Wiederholen des Verfahrens, bis die gewünschte Konzentration erreicht ist.

24. Verfahren nach Anspruch 22 oder Anspruch 23, wobei die Methode des Weiteren umfasst:
Füllen der Kammer der zweiten Mischpumpe (700) mit einem vorbestimmten Volumen von zweiter Dialysat-Basislösung und Wasser;
Pumpen eines Anteils der Mischung der zweiten Dialysat-Basislösung und Wasser, und ein vorbestimmtes Volumen von erster Dialysatlösung-Basis in die Kammer der ersten Mischpumpe (720);
Einbehalten eines Anteils der Mischung von zweiter Dialysat-Basislösung und Wasser in der Kammer der zweiten Mischpumpe (700).

25. Verfahren nach Anspruch 24, wobei das Verfahren des Weiteren umfasst:
Messen einer Eigenschaft der aus der zweiten Mischkammer austretenden zweiten Dialysat-Basislösung und Wasser, um ein deren Konzentration anzeigendes Signal zu generieren;
Bestimmen, ob die Konzentration größer oder geringer ist als die benötigte Konzentration;
Steuern der zweiten Dialysatlösung-Basis-Pumpe (708; 808; 908), um die Menge der zur Kammer der zweiten Mischpumpe (720; 800; 900) hinzugefügten zweiten Dialysatlösung-Basis entsprechend zu erhöhen oder zu erniedrigen.

26. Verfahren nach einem der Ansprüche 22 bis 25, wobei das Verfahren umfasst:
Wiederholen der Schritte des Füllens und teilweise Leerens der Kammern, bis ein Gleichgewicht erreicht ist und die benötigte Dialysatkonzentration erreicht ist.

27. Einsatz (10) nach einem der Ansprüche 1 bis 21, wobei der Einsatz (10) des Weiteren eine Blut-Flussbahn umfasst, um ein Volumen zu behandelnden Blutes im Dialysator zu transportieren, oder wobei der Einsatz (10) ein Einwegeinsatz ist.

## Revendications

1. Cartouche (10) destinée à être utilisée dans une machine d'hémodialyse (1), ladite cartouche (10) comprenant :
un trajet d'écoulement de dialysat comprenant un dialyseur (80), ledit trajet d'écoulement de dialysat étant destiné à fournir un écoulement de dialysat audit dialyseur (80);
une première pompe de mélange (600) qui comprend une chambre ayant un volume fixe entre un renfoncement concave et une membrane flexible (22), ladite chambre étant destinée à recevoir un volume prédéterminé d'une première base de solution de dialysat, et un volume d'eau ;
une pompe à équilibrage de débit (640) qui comprend : une chambre ayant un volume fixe entre un renfoncement concave et une membrane flexible (22), et une admission par laquelle elle reçoit ledit dialysat ; un trajet d'écoulement de fluide qui relie ladite première pompe de mélange (600) à ladite pompe à équilibrage de débit (640) ;
ladite cartouche (10) comprenant en outre:
un premier clapet de non-retour (650, 654) ayant une admission en communication de fluide avec ledit trajet d'écoulement de fluide, ledit clapet de non-retour (650, 654) étant configuré pour s'ouvrir si la pression dans ledit trajet d'écoulement de fluide dépasse une pression prédéterminée, afin de permettre à l'excédent de fluide situé dans ledit trajet d'écoulement de fluide de s'écouler par ledit premier clapet de non-retour (650, 654) vers une purge (652), et de se fermer lorsque la pression dans ledit trajet d'écoulement de fluide devient inférieure à ladite pression prédéterminée.

2. Cartouche (10) selon la revendication 1, qui comprend en outre:
une seconde pompe de mélange (620) en aval de ladite première pompe de mélange (600), ladite seconde pompe de mélange (620) comprenant une chambre ayant un volume fixe entre un renfoncement concave et une membrane flexible (22), destinée à recevoir un volume prédéterminé d'une seconde base de solution de dialysat et le mélange d'eau et de ladite première base de solution de dialysat de la part de ladite première pompe de mélange (620), et dans laquelle
l'admission dudit premier clapet anti-retour (650) est en communication de fluide avec ledit trajet d'écoulement de fluide entre lesdites première et seconde pompes de mélange.

3. Cartouche (10) selon la revendication 2, qui comprend en outre:
un second clapet anti-retour (654) ayant une admission, ladite admission dudit second clapet de non-retour étant en communication de fluide avec ledit trajet d'écoulement de fluide entre ladite seconde pompe de mélange (620) et ladite pompe d'équilibrage (640), dans laquelle
ledit second clapet de non-retour (654) est configuré pour s'ouvrir si la pression dans ledit trajet d'écoulement de fluide entre ladite seconde pompe de mélange (620) et ladite pompe d'équilibrage (640) augmente au-delà d'une pression prédéterminée afin de permettre à l'excédent de fluide dans ledit trajet d'écoulement de fluide de circuler par ledit second clapet de non-retour (654) vers une purge (656), et de se fermer lorsque la pression dans ledit trajet d'écoulement de fluide devient inférieure à ladite pression prédéterminée.

4. Cartouche (10) selon la revendication 2, dans laquelle ladite chambre de ladite seconde pompe de mélange (620) et ladite chambre de ladite pompe d'équilibrage de débit (640) sont une seule et même chambre.

5. Cartouche (10) selon la revendication 1, qui comprend en outre:
une seconde pompe de mélange (620) en aval de ladite première pompe de mélange (600), ladite seconde pompe de mélange (620) comprenant une chambre ayant un volume fixe entre un renfoncement concave et une membrane flexible (22), ladite chambre étant destinée à recevoir un volume prédéterminé d'une seconde base de solution de dialysat et le mélange d'eau et de première base de solution de dialysat de la part de ladite première pompe de mélange (600), et dans laquelle
l'admission dudit premier clapet anti-retour (654) est en communication de fluide avec ledit trajet d'écoulement de fluide entre ladite seconde pompe de mélange (620) et ladite pompe d'équilibrage de débit (640).

6. Cartouche (10) selon la revendication 5, dans laquelle ladite seconde pompe de mélange (620) possède un volume supérieur à celui de ladite première pompe de mélange (600).

7. Cartouche (10) selon la revendication 1, qui comprend en outre:
une première pompe à base de dialysat (608), ladite première pompe à base de dialysat (608) est configurée pour ajouter un volume de première base de dialysat à ladite première pompe de mélange (600), et
un contrôleur (610) destiné à contrôler ladite première pompe à base de dialysat (608).

8. Cartouche (10) selon la revendication 7, qui comprend en outre:
un premier capteur (616) dans ledit trajet d'écoulement de fluide, ledit capteur (616) étant en aval de ladite première pompe de mélange (600), le capteur (616) étant prévu pour fournir un signal qui indique la concentration en mélange de première base de solution de dialysat et d'eau qui quitte ladite première pompe de mélange (600) audit contrôleur (610), et dans laquelle
ledit contrôleur (610) est configuré pour contrôler ladite première pompe à base de solution de dialysat (608) afin d'obtenir un mélange ratiométrique prédéterminé de première base de solution de dialysat et d'eau.

9. Cartouche (10) selon la revendication 7 ou 8, qui comprend en outre:
un premier capteur de base de solution de dialysat (660) qui crée un signa qui indique la concentration en première base de solution de dialysat, et dans laquelle
ledit contrôleur (610) reçoit ledit signal et l'utilise pour contrôler ladite première pompe à base de solution de dialysat (608) afin d'obtenir un mélange ratiométrique prédéterminé de première base de solution de dialysat et d'eau dans ladite chambre de ladite première pompe de mélange (600).

10. Cartouche (10) selon la revendication 2, qui comprend en outre:
une seconde pompe à base de dialysat (626) configurée pour ajouter un volume de seconde base de dialysat à ladite seconde pompe de mélange (620), et
un contrôleur (627) destiné à contrôler ladite seconde pompe à base de dialysat (626).

11. Cartouche (10) selon la revendication 10, qui comprend en outre:
un capteur (634) en aval de ladite seconde pompe de mélange (620), qui crée un signal qui indique la concentration en mélange de seconde base de solution de dialysat et en mélange d'eau et de première base de solution de dialysat qui quitte ladite seconde pompe de mélange (620), et dans lequel
ledit contrôleur (627) reçoit ledit signal et l'utilise pour contrôler ladite seconde pompe à base de solution de dialysat (620) afin d'obtenir un mélange ratiométrique prédéterminé de seconde base de solution de dialysat et de mélange d'eau et de première solution de dialysat.

12. Cartouche (10) selon la revendication 10 ou 11, qui comprend en outre :
un second capteur de base de solution de dialysat qui crée un signal qui indique la concentration en seconde base de solution de dialysat, et dans laquelle
ledit contrôleur (627) reçoit ledit signal et l'utilise pour contrôler ladite seconde pompe à base de solution de dialysat (620) afin d'obtenir un mélange ratiométrique prédéterminé de seconde base de solution de dialysat et de mélange d'eau et de première solution de dialysat dans ladite chambre de ladite seconde pompe de mélange (620).

13. Cartouche (10) destinée à être utilisée dans une machine d'hémodialyse (1),ladite cartouche (10) comprenant :
un trajet d'écoulement de dialysat qui comprend un dialyseur, ledit trajet d'écoulement de dialysat étant destiné à fournir un écoulement de dialysat audit dialyseur ;
une première pompe de mélange (720 ; 800 ; 900) qui comprend une chambre ayant un volume fixe entre un renfoncement et une membrane flexible (22), ladite chambre étant destinée à recevoir un volume prédéterminé d'une première base de solution de dialysat, et un volume d'eau ;
une première pompe à base de solution de dialysat (708 ; 808 ; 908), contrôlée par un contrôleur (710 ; 810 ; 910), et destinée à ajouter un volume prédéterminé d'une première base de solution de dialysat à ladite chambre de ladite première pompe de mélange (720 ; 800 ; 900) ;
une pompe d'équilibrage de débit (740 ; 840 ; 940) qui comprend une chambre ayant un volume fixe entre un renfoncement et une membrane flexible (22), et une admission par laquelle elle reçoit ledit dialysat ; et
un trajet d'écoulement de fluide qui relie ladite première pompe de mélange (720 ; 800 ; 900) à ladite pompe d'équilibrage de débit (740 ; 840 ; 940) ;
dans laquelle le volume minimum autorisé de ladite première pompe de mélange (720 ; 800 ; 900) est égal, ou supérieur, au volume maximum autorisé de ladite pompe d'équilibrage (740 ; 840 ; 940).

14. Cartouche (10) selon la revendication 13, qui comprend en outre :
une seconde pompe de mélange de dialysat (700) en amont de ladite première pompe de mélange de dialysat (720), ladite seconde pompe de mélange (700) comprenant une chambre ayant un volume fixe entre un renfoncement et une membrane flexible (22), ladite chambre étant destinée à recevoir un volume prédéterminé d'une seconde base de solution de dialysat, et un volume d'eau ;
une seconde pompe de base de solution de dialysat (726), contrôlée par un contrôleur (727), et destinée à ajouter un volume prédéterminé d'une seconde base de solution de dialysat à ladite chambre de ladite seconde pompe de mélange (700) ; et
un second trajet d'écoulement de fluide qui relie lesdites seconde (700) et première (720) pompes de mélange afin de fournir un mélange d'eau et de seconde solution de base de dialysat à ladite chambre de ladite première pompe de mélange (720).

15. Cartouche (10) selon la revendication 14, dans laquelle :
ledit volume minimum autorisé de ladite seconde pompe de mélange (700) est égal, ou supérieur, au volume maximum autorisé de ladite première pompe de mélange (720).

16. Cartouche (10) selon la revendication 13 ou 14, dans laquelle la cartouche (10) comprend en outre :
un capteur (734) en aval de ladite première pompe de mélange (720), ledit capteur (734) étant configuré pour créer un signal qui indique la concentration en fluide qui quitte ladite première pompe de mélange (720).

17. Cartouche (10) selon la revendication 14, où la cartouche (10) comprend en outre :
un capteur (716) en aval de ladite seconde pompe de mélange (700), ledit capteur (716) étant configuré pour créer un signal qui indique la concentration en fluide qui quitte ladite seconde pompe de mélange (700).

18. Cartouche (10) selon la revendication 13, dans laquelle :
ladite pompe d'équilibrage (840 ; 940) est configurée pour recevoir, dans sa chambre : un mélange d'eau et de première solution de base de dialysat de la part de la chambre de ladite première pompe de mélange (800 ; 900), et une seconde solution de base de dialysat de la part d'une seconde pompe à base de solution de dialysat (826 ; 926).

19. Cartouche (10) selon la revendication 18, qui comprend en outre :
un capteur (816 ; 916) en aval de ladite pompe d'équilibrage (800 ; 900), ledit capteur (816 ; 916) étant configuré pour créer un signal qui indique la concentration en fluide qui quitte ladite chambre de ladite pompe d'équilibrage (800 ; 900).

20. Cartouche (10) selon la revendication 16, 17 ou 19, dans laquelle, pendant l'utilisation, des signaux de retour des capteurs (734 ; 716 ; 916) sont fournis aux contrôleurs respectifs (710 ; 727 ; 910) desdites première et seconde pompes à base de solution de dialysat, lesdits contrôleurs respectifs étant configurés pour contrôler lesdites première (708 ; 908) et seconde (726 ; 926) pompes de base de solution de dialysat afin de modifier la quantité de première et de seconde bases de solution de dialysat qu'elles ajoutent auxdites première et seconde pompes de mélange (720 ; 700), ou à ladite première pompe de mélange (900) et à ladite pompe d'équilibrage (740 ; 940), respectivement, de façon à obtenir un mélange ratiométrique prédéterminé de première et de seconde bases de solution de dialysat et d'eau.

21. Cartouche (10) selon la revendication 16, 17 ou 19, dans laquelle
lors de l'utilisation, les signaux provenant desdits capteurs (734 ; 716 ; 916) sont surveillés et provoquent une alarme si le signal généré se trouve en-dehors de paramètres spécifiques.

22. Procédé de mélange d'une solution de dialysat dabs ladite cartouche (10) selon l'une quelconque des revendications 13 à 21, ledit procédé comprenant :
le remplissage de la chambre de ladite première pompe de mélange (720 ; 800 ; 900) avec un volume prédéterminé de première solution de base de dialysat et d'eau ;
le pompage d'une proportion dudit mélange dans ladite chambre de ladite première pompe de mélange (720 ; 800 ; 900) dans ladite chambre de ladite pompe d'équilibrage (740 ; 840 ; 940) ; et
la conservation d'une proportion dudit mélange de ladite première solution de base de dialysat et d'eau dans ladite chambre de ladite première pompe de mélange (720 ; 800 ; 900).

23. Procédé selon la revendication 22, qui comprend en outre :
la mesure d'une propriété de ladite solution de base de dialysat et d'eau quittant ladite première chambre de mélange (720 ; 800 ; 900) afin de générer un signal qui indique sa concentration,
la détermination du fait que ladite concentration soit supérieure ou inférieure à la concentration requise ;
le contrôle de ladite première pompe de base de solution de dialysat (708 ; 808 ; 908) afin d'augmenter ou de diminuer la quantité de première base de solution de dialysat ajoutée à ladite chambre de ladite première pompe de mélange (720 ; 800 ; 900) en conséquence, et la répétition dudit procédé jusqu'à ce que la concentration requise soit atteinte.

24. Procédé selon la revendication 22 ou 23, dans laquelle ledit procédé comprend en outre :
le remplissage de ladite chambre de ladite seconde pompe de mélange (700) avec un volume prédéterminé de seconde solution de base de dialysat et d'eau ;
le pompage d'une proportion dudit mélange de seconde solution de base de dialysat et d'eau, et d'un volume prédéterminé de première base de solution de dialysat, dans la chambre de ladite première pompe de mélange (720) ;
la conservation d'une proportion dudit mélange de seconde solution de base de dialysat et d'eau dans la chambre de ladite seconde pompe de mélange (700).

25. Procédé selon la revendication 24, qui comprend en outre :
la mesure d'une propriété de ladite seconde solution de base de dialysat et d'eau qui quitte ladite seconde chambre de mélange, afin de générer un signal qui indique sa concentration,
la détermination du fait que ladite concentration soit supérieure ou inférieure à la concentration requise ;
le contrôle de la seconde pompe de base de solution de dialysat (726) afin d'augmenter ou de diminuer la quantité de seconde base de solution de dialysat ajoutée à ladite chambre de ladite seconde pompe de mélange (700) en conséquence.

26. Procédé selon l'une quelconque des revendications 22 à 25, qui comprend :
la répétition des étapes de remplissage et de visage partiel desdites chambres jusqu'à ce qu'un équilibre soit atteint et que les concentrations en dialysat requises soient obtenues.

27. Cartouche (10) selon l'une quelconque des revendications 1 à 21, où la cartouche (10) comprend en outre un trajet d'écoulement du sang destiné à acheminer un volume de sang à traiter dans ledit dialyseur, ou qui est jetable.
